# EUROPEAN PATENT APPLICATION

(11) **EP 3 031 820 A1**
(43) Date of publication of application: **15.06.2016**
(21) Application number: 14196845.3
(22) Date of filing: 08.12.2014
(51) Int. Cl.: C07K 14/005, C07K 14/01, C12N 7/04

(54) **JC Polyomavirus VLP (virus-like particle) with a targeting peptide**

(71) Applicant: Life Science Inkubator, 53175 Bonn (DE)
(72) Inventor: Franken, Sebastian, 53175 Bonn (DE); Glassmann, Alexander, 53175 Bonn (DE); Temme, Nadine, 53175 Bonn (DE)
(74) Representative: von Renesse, Dorothea

(57) **Abstract**

The disclosure relates to a fusion protein comprising at least a first and a second peptide, wherein
- the second peptide comprises a targeting region and a first and a second interaction region,
- the second peptide is located on the surface of the fusion protein;
- the second peptide comprises at least two interaction pairs, wherein an interaction pair is formed by an amino acid of the first interaction region and an amino acid of the second interaction region,
- the interaction between the amino acids of an interaction pair is covalent or noncovalent; and
- at least one interaction pair is a covalent interaction pair in which the amino acids are covalently bound,

and to virus like particles (VLP) comprising the fusion protein for use as drug delivery system.

Also provided are polynucleotides encoding the fusion protein, suitable expression vectors, host cells, production methods for the fusion protein and the VLP comprising the fusion protein.

## Description

### FIELD OF THE INVENTION

The present invention relates to a fusion protein comprising a peptide with a targeting region and a first and a second interaction region for targeting the fusion protein and to virus like particles (VLP) comprising the fusion protein for use as drug delivery system.

### BACKGROUND OF THE INVENTION

In the development of specific diagnostic or therapeutic procedures, the use of transfer systems (delivery systems) that allow a possible cell-specific transfer of substances and nucleic acids such as, markers or agents, is of great importance. For this cell-specific transfer systems based on virus-like particles (VLP) have been developed. One of these systems is the VLP from the human polyomavirus John-Cunningham virus (JCV) as described in WO 97/19174 and EP 1270586 B1. Basis of this system is the ability of the VLP to package foreign cargo such as drugs or and nucleic acids instead of the viral DNA. As a VLP still has the ability to specifically recognize cells and to be internalized by the cells the VLP can be used to introduce a cargo of choice into specific cells. However, the VLPs of the state art interact with a very broad spectrum of cells. For certain applications it is desirable to be able to address only specific cell types.

In view of this prior art it is an object of the present invention to improve VLPs for the use as a drug delivery system. In particular it is an object of the present invention to provide means to improve the cell specificity of VLPs.

### SUMMARY OF THE INVENTION

According to a first aspect, the invention provides a fusion protein comprising at least a first and a second peptide, wherein
- the second peptide comprises a targeting region and a first and a second interaction region,
- the second peptide is located on the surface of the fusion protein,
- the second peptide comprises at least two interaction pairs, wherein an interaction pair is formed by an amino acid of the first interaction region and an amino acid of the second interaction region,
- the interaction region between the amino acid of an interaction pair is covalent or non-covalent, and
- at least one interaction pair is a covalent interaction pair in which the amino acids are covalently bound.

The second peptide according to the invention is a modular targeting peptide that has several advantages for targeting a protein of interest to which it is fused forming the fusion protein. The second peptide provides a self-forming secondary structure that presents a specific targeting sequence of choice in form of a loop spaced apart from the surface of the fusion protein. Due to the spacing of the targeting sequence away from the surface of the fusion protein, the second peptide provides an improved accessibility of the targeting sequence. The fusion protein may be used in particular in combination with a virus-like particle (VLP) cargo transport to provide to the VLP the ability to target specific predefined receptors/cell types.

Thus, according to the second aspect, the invention provides a VLP comprising the fusion peptide of the first aspect of the invention.

According to a third aspect, the invention provides a pharmaceutical composition comprising the VLP according to the second aspect and at least one pharmaceutically acceptable carrier.

According to a fourth aspect, the invention provides an isolated polynucleotide comprising a nucleic acid sequence encoding a fusion protein according to the first aspect of the invention. According to a fifth aspect, the invention provides an expression vector comprising the nucleotide according to the fourth aspect of the invention.

According to a sixth aspect, the invention provides a host cell comprising the expression vector according to the fifth aspect of the invention.

According to a seventh aspect, the invention provides a process of producing the VLP according to the second aspect of the invention which comprises the steps of:
a) introducing a polynucleotide according to the second aspect of the invention into a host cell;
b) culturing the transformed host cell in a medium under conditions leading to a protein expression with the polynucleotide as a template,
c) isolating the expression product from the cell and
d) assembly of the expression product optionally with further viral proteins into the VLP

### BRIEF DESCRIPTION OF THE FIGURES

- **Fig. 1**: shows a specific embodiment of the second peptide according to the invention
- **Fig. 2**: provides an overview of a specific embodiment for the production of VLPs comprising a fusion protein according to the invention.
- **Fig. 3**: shows histograms calculated from flow cytometry data obtained from MDA-MB-231 cells treated with VLPs. The histograms a) to c) correspond to the following VLPs:
a) VLP with VP1 and VP1-FITC, VLP with VP1-DE-Loop-Lyp1 and VP1-FITC, VLP with VP1-HI-Loop-Lyp1 and VP1-FITC
b) VLP with VP1 and VP1-Dylight488, VLP with VP1-DE-Loop-Lyp1 and VP1-Dylight488, VLP with VP1-HI-Loop-Lyp1 and VP1-Dylight488
c) VLP with VP1 and VP1-Atto647, VLP with VP1-DE-Loop-Lyp1 and VP1-Atto647, VLP with VP1-HI-Loop-Lyp1 and VP1-Atto647.
   The X-axis represents the measured intensity of the signal, the Y-axis the number of cells with the specific signal intensity.

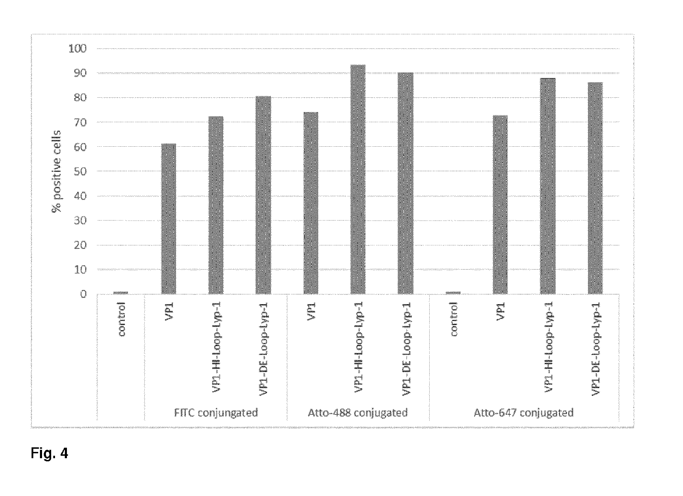

- **Fig. 4**: shows a column diagram representation of the flow cytometry data obtained from MDA-MB-231 cells treated with VLPs. Each column represents the percentage of cells - identified by a minimum signal strength - with a positive uptake of a specific type combination of VLP and fluorescent dye as indicated below the columns. Three different VLPs are tested: VLPs containing VP1-DE-Loop-Lyp1 or VP1-HI-Loop-Lyp1 VLPs from normal VP1. Also three different dyes are tested: FITC, Atto-488 and Atto 647.

### DETAILED DESCRIPTION OF THE INVENTION

### 1. Definitions

A "peptide" according to the present invention may be composed of any number of amino acids of any type, preferably naturally occurring amino acids, which preferably are linked by peptide bonds. In particular, a peptide comprises at least 3 amino acids, preferably at least 5, at least 7, at least 9, at least 12 or at least 15 amino acids. Furthermore, there is no upper limit for the length of a peptide. However, preferably a peptide according to the invention does not exceed a length of 500 amino acids, more preferably, it does not exceed a length of 300 amino acids; even more preferably, it is not longer than 250 amino acids. Thus, the term peptide includes oligopeptides, which usually refer to peptides with a length of 2 to 10 amino acids, and polypeptides, which usually refer to peptides with a length of more than 10 amino acids. The term "protein" refers to a peptide with at least 60, at least 80, preferably at least 100 amino acids.

The term "fusion protein" according to the invention relates to proteins or peptides created through the joining of two or more genes that originally coded for separate proteins. The genes may be naturally occurring from the same organism or different organisms or may be synthetic polynucleotides.

The term "exogenous" according to the invention relates to the property of a peptide or polynucleotide that it does not naturally occur in polyomaviruses.

The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et a/., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

### (Identical Residues x 100)/(Length of Alignment - Total Number of Gaps in Alignment)

For purposes of the present invention, the degree of sequence identity between two deoxyribonucleotide sequences is determined using the Needleman-Wunsch algorithm (Needleman and Wunsch, 1970, supra) as implemented in the Needle program of the EMBOSS package (EMBOSS: The European Molecular Biology Open Software Suite, Rice et a/., 2000, supra), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

### (Identical Desoxyribonucleotides x 100)/(Length of Alignment - Total Number of Gaps in Alignment)

The term "isolated" means a substance in a form or environment which does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature.

The term "operably linked" means a configuration in which a control sequence is placed at an appropriate position relative to the coding sequence of a polynucleotide such that the control sequence directs the expression of the coding sequence. Expression: The term "expression" includes any step involved in the production of the polypeptide including, but not limited to, transcription, post-transcriptional modification, translation, post-translational modification, and secretion.

The term "expression vector" means a linear or circular DNA molecule that comprises a polynucleotide encoding a polypeptide and is operably linked to additional nucleotides that provide for its expression.

The term "host cell" means any cell type that is susceptible to transformation, transfection, transduction, and the like, with a nucleic acid construct or expression vector comprising a polynucleotide of the present invention. The term "host cell" encompasses any progeny of a parent cell that is not identical to the parent cell due to mutations that occur during replication.

The term "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The term "carrier" applied to pharmaceutical compositions of the invention refers to a diluent, excipient, or vehicle with which the VLP of the invention is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington The Science and Practice of Pharmacy," 21th edition, (David B. Troy ed., 2006, p. 745-775, p. 802-836 and p. 837 - 849).

As used herein, the term "pharmaceutical composition" refers to any composition comprising at least the VLP with or without cargo and at least one other ingredient, as well as any product which results, directly or indirectly, from combination, complexation, or aggregation of any two or more of the ingredients, from dissociation of one or more of the ingredients, or from other types of reactions or interactions of one or more of the ingredients. Accordingly, the term "pharmaceutical composition" as used herein may encompass, inter alia, any composition made by admixing a pharmaceutically active ingredient and one or more pharmaceutically acceptable carriers.

The term "low stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 25 percent formamide, following standard Southern blotting procedures for 12 to 24 hours. The carrier material is finally washed three times each for 15 minutes using 2x SSC, 0.2 percent SDS at 50 degrees centigrade.

The term "medium stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 35 percent formamide, following standard Southern blotting procedures for 12 to 24 hours. The carrier material is finally washed three times each for 15 minutes using 2x SSC, 0.2 % SDS at 55 °C.

The term "high stringency conditions" means for probes of at least 100 nucleotides in length, prehybridization and hybridization at 42 degrees centigrade in 5x SSPE, 0.3 percent SDS, 200 micrograms/ml sheared and denatured salmon sperm DNA, and 50 percent formamide, following standard Southern blotting procedures for 12 to 24 hours. The carrier material is finally washed three times each for 15 minutes using 2x SSC, 0.2 % SDS at 65 °C.

The term "PBS" means phosphate buffered saline. It is a water-based salt solution containing sodium phosphate, sodium chloride and, in some formulations, potassium chloride and potassium phosphate. The osmolarity and ion concentrations of the solutions match those of the human body.

### 2. Fusion Protein

According to a first aspect, the invention provides a fusion protein comprising at least a first and a second peptide, wherein:
- the second peptide comprises a targeting region and a first and a second interaction region,
- the second peptide is located on the surface of the fusion protein,
- the second peptide comprises at least two interaction pairs, wherein an interaction pair is formed by an amino acid of the first interaction region and an amino acid of the second interaction region,
- the interaction region between the amino acid of an interaction pair is covalent or non-covalent, and
- at least one interaction pair is a covalent interaction pair in which the amino acids are covalently bound.

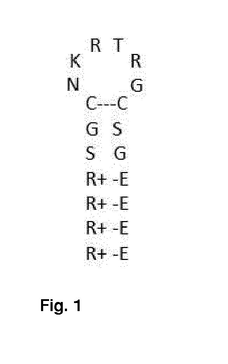

The second peptide according to the invention, i.e. the targeting peptide, is based on a particular secondary structure resembling a hair pin known from single-stranded polynucleotides especially in RNA molecules. When folded into its secondary structure, the peptide preferably comprises two paired regions of the amino acid sequence, the first and second interaction region and an unpaired loop comprising the targeting region as shown schematically in **Fig. 1**.

The targeting region according to the invention comprises an amino acid sequence - the targeting sequence - that is known to interact with a target of interest, in particular a cellular receptor. The secondary structure of the second peptide may also be described as a stem loop comprising a stem region and a loop region. Accordingly, the two interaction regions of the peptide preferably form the stem and the targeting region forms the loop (see **Fig. 1**). When located on the surface of the fusion protein, the stem, i.e. the first and second interaction region of the second peptide, lead to a sufficient spacing between the surface of the protein and the targeting region so that an interaction with a targeting recognizing means, in particular a cellular receptor, is possible without steric hindrance.

The folding of the structure is based on the following theoretic principle. During protein folding, the amino acids on the first and second interaction region get into proximity. When two complementary amino acids of the two interaction regions get in proximity to each other, they will transiently bind to each other, i.e. interact non-covalently, and, thus, form a non-covalent interaction pair. The more interaction pairs are formed at a time, the higher is the binding strength and the longer the transient interaction of the two interaction regions. The interaction pairs of the second peptide are preferably set up such that the formation of these non-covalent interaction pairs brings the amino acids of the covalent interaction pair, in particular cysteines, into proximity to each other for a sufficient time so as to allow formation of a covalent bond, e.g. a disulfide-bridge. The formation of the covalent interaction pair leads to a further stabilization of the interaction of the first and second interaction region of the second peptide. Accordingly, the final secondary structure of the second peptide with a loop including the targeting region and a stem formed by the first and second interaction region is formed. The loop can be regarded as a circular peptide connected by a covalent interaction pair. It was shown for a variety of signaling/targeting peptides that a circular shape of the peptide improves its recognition by the specific receptor.

Thus, according to one embodiment of the first aspect of the invention, the amino acid sequence of the targeting region is located between the amino acid sequences of the first and second interaction region. A location of the amino acid sequence of the targeting region between the first and second interaction region is required to obtain a targeting region that is located in the loop of the folded second peptide.

The amino acid sequence of the targeting region may overlap with the amino acid sequences of the first and/or second interaction region. In particular, the amino acids forming the covalent interaction pair may be part of the targeting region.

The amino acid sequence of the targeting region may be any sequence that is recognized or binds to a target molecule, in particular a cellular receptor. Non-limiting examples of such peptides are Lyp-1 (SEQ ID NO: 1), RGD, RGR, HER2 binding peptide (SEQ ID NO: 2), CREKA peptide (SEQ ID NO: 3), NGR peptide, CPP-2 (SEQ ID NO: 4), CPP-44 (SEQ ID NO: 5), F3 (SEQ ID NO: 6), RMS-P3 (SEQ ID NO: 7), F56 (SEQ ID NO: 8), LTVSPWY-peptide (SEQ ID NO: 9), WNLPWYYSVSPT-peptide (SEQ ID NO: 10), and SP5-2 (SEQ ID NO: 11).

Thus, according to one embodiment of the first aspect of the invention, the targeting region comprises a sequence selected from the group consisting of SEQ ID NO: 1, RGD, RGR, SEQ ID NO: 2, SEQ ID NO: 3, NGR peptide, SEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11. Preferably the amino acid sequence of the targeting region comprises SEQ ID NO: 1.

Lyp-1 is a tumor homing peptide that selectively binds the tumor-associated lymphatic vessels and tumor cells in certain tumors. The nine amino acid long peptide specifically recognizes the receptor P32. The RGD-peptide and NGR-peptide are tri-peptides composed of L-arginine-glycine-L-aspartic acid and L-asparagine-glycine-L-arginine, respectively. The sequences are common elements in cellular recognition. RGD peptides are implicated in cellular attachment via integrins. The HER2 binding peptide specifically targets the Human Epidermal Growth Receptor 2 (HER2). The CREKA peptide is a tumor homing peptide identified in phage display libraries consisting of the sequence Cys-Arg-Glu-Lys-Ala (see Simberg D, et al. Biomimetic amplification of nanoparticle homing to tumors. Proc Natl Acad Sci USA. 2007 Jan 16;104(3):932-6). CPP-2 and CPP-44 are tumor homing peptides described in Kondo et al. Tumourlineage-homing cell-penetrating peptides as anticancer molecular delivery systems. Nat Commun. 2012 Jul 17;3:951. F3 comprises amino acid sequences 17-48 of High Mobility Group Nucleosomal Binding Protein 2 (HMGN2) and was identified in a phage display cDNA library screen for peptides capable of homing to tumors, especially to their vascular endothelium (see (see Christian et al., Nucleolin expressed at the cell surface is a marker of endothelial cells in angiogenic blood vessels. J Cell Biol. 2003 Nov 24;163(4):871-8). RMS-P3 is a furin targeted peptide suitable for targeting Rhabdomyosarcoma (RMS) cells (see Hajdin K, et al. Furin targeted drug delivery for treatment of rhabdomyosarcoma in a mouse model. PLoS One. 2010 May 3;5(5)). F56 specifically binds to VEGF receptor Flt-1 (see Herringson and Altin, Effective tumor targeting and enhanced anti-tumor effect of liposomes engrafted with peptides specific for tumor lymphatics and vasculature. Int J Pharm. 2011 Jun 15;411(1-2):206-14). LTVSPWY-peptide and WNLPWYYSVSPT-peptide specifically bind to breast cancer cells (see Shadidi and Sioud, Identification of novel carrier peptides for the specific delivery of therapeutics into cancer cells, FASEB J. 2003 Feb;17(2):256-8). SP5-2 specifically binds to non-small cell lung cancer (see Chang DK, et al. A Novel Peptide Enhances Therapeutic Efficacy of Liposomal Anti-Cancer Drugs in Mice Models of HumanLung Cancer, PLoS ONE 2009 (1):e4171).

According to one embodiment of the first aspect of the invention, the loop between the first and second interaction region which comprises the targeting region has a number of amino acids in the range from 3 to 50 amino acids. The number of amino acids of the loop is counted from the covalent interaction pair "closing" the loop and consequently includes the amino acids of the covalent interaction pair. Accordingly a number of loop amino acids of 2 only includes the covalent interaction pair. Thus, the minimal number of amino acids in the loop is 3. The maximum length of the loop is in principle limited by the influence of the peptide on the folding of the fusion protein and the tendency for aggregation with higher length. Thus, the maximum number of amino acids in the loop is preferably 25, more preferably 20, most preferably 15 amino acids. According to a particularly preferred embodiment, the number of amino acids in the loop is in the range from 5 to 15 amino acids.

The covalent interaction pair may be formed by any two amino acids, the side chains of which may form a covalent bond. These may be in particular cysteines or seleno cysteines which form disulfide bridges. According to one embodiment of the first aspect of the invention, the covalent interaction pair is formed by a cysteine in the first interaction region and by a cysteine in the second interaction region. A fusion protein according to the invention may comprise more than one covalent interaction pair. For example, the fusion protein according to the invention may comprise 7 or less, 6 or less, 5, or less, 4 or less, 3 or less, 2 or less interaction pairs. The covalent interaction pairs may be located in sequence or spaced apart. Preferably, the fusion protein according to the invention comprises one covalent interaction pair.

According to one embodiment of the first aspect of the invention, at least two interaction pairs are non-covalent interaction pairs in which the amino acids interact non-covalently. Preferably, the second peptide comprises at least 3 non-covalent interaction pairs, more preferably at least 4 non-covalent interaction pairs. In principal, the higher the number of interaction pairs, the stronger the interaction of the first and second interaction region of the second peptide. The number of non-covalent interaction pairs also depends on the type of interaction of the amino acids. The non-covalent interaction may be by hydrogen bridges, van der Waal forces, hydrophobic interactions or acid-base interactions.

Preferably, at least a part of the non-covalent interaction pairs are acid-base interaction pairs formed by an acidic amino acid in one interaction region and a basic amino acid in the other interaction region. At a neutral pH, these amino acids are charged negatively and positively, respectively. The contrary charges of the amino acids lead to an attraction of these amino acids and consequently of the interaction regions. Moreover, the contrary charges provide a tight binding of binding.

According to one embodiment, the second peptide comprises 2 to 20 acid-base interaction pairs, preferably 2 to 10 acid-base interaction pairs, more preferably 2 to 6 acid-base interaction pairs, most preferably 3 to 5 acid-base interaction pairs. The higher the number of acid-base interaction pairs, the higher the attraction of the first and second interaction region. However, a number more than 20 acid-base interaction pairs will be problematic for the folding of the fusion protein. A number of more than 10 acid-base interaction pairs renders cloning more problematic as very long primers have to be used. Moreover, it is assumed that the use of more than 6 acid-base interaction pairs does not further significantly increase the interaction of the first and second interaction region. With regard to ease of cloning and optimal strength of interaction of the non-covalent interaction pairs, a number of 3 to 5 acid-base interaction pairs are preferred. In a particularly preferred embodiment of the first aspect of the invention the second peptide comprises 4 acid-base interaction pairs.

Basic amino acids according to the invention can be arginine, lysine or histidine. Acidic amino acids according to the invention can be glutamic acid or aspartic acid.

The first and second interaction region may comprise both acidic and basic amino acids, only acidic amino acids or only basic amino acids. The basic and acid amino acids in one interaction region may be alternating or form clusters. Non-limiting examples of alternating sequences are: EERR, ERER, EERREE, RREERR. Examples of clusters are EEERRR, DDERKK, DDDRR. However, it is preferred that one of the interaction regions comprises a majority of acidic amino acids and the other, consequently, a majority of basic amino acids. For example, the first inter action region comprises the mainly basic sequence RRRRE and the second interaction region comprises the mainly acidic sequence EEEER.

According to a preferred embodiment of the first aspect of the invention, the non-covalent interaction pairs are acid-base interaction pairs and formed by an acidic amino acid in the first interaction region and basic amino acid in the second region. The first interaction region may comprise at least 2, at least 3, at least 4, at least 5, at least 6 acidic amino acids. Also, the second interaction region may comprise at least 2, at least 3, at least 4, at least 5, at least 6 basic amino acids. Preferably, the first interaction region comprises at least 4 acidic amino acids and the second interaction region comprises at least 4 basic amino acids. More preferably, the first interaction region comprises at least 4 consecutive acidic amino acids and the second interaction region comprises at least 4 consecutive basic amino acids.

According to one embodiment of the first aspect of the invention, the majority of the basic amino acids are arginine. In an alternative embodiment, the majority of the basic amino acids are lysines. Preferably, all basic amino acids in the interaction region are arginines.

The charged amino acids in the interaction regions, i.e. the acidic and basic amino acids of the first and second interaction region may be directly in sequence or contain non-charged amino acids as spacers in between. Accordingly, two charged amino acids in the interaction region may be directly connected or may be separated by one or more non-charged amino acids. The non-charged amino acids as spacer between the charged amino acids are preferably selected from the group consisting of serine and glycine. The number of non-charged amino acids, i.e. the spacing, between two charged amino acids may be for example 0, 1, 2, 3 or 4. According to one embodiment of the invention, the spacing of the charged amino acids within the amino acid sequence of the first interaction region is 0 or 1. According to one embodiment of the invention the spacing of the charged amino acids in the second interaction region is 0 or 1. According to one embodiment the spacing of the charged amino acids in the first and/or second interaction region is preferably 0. Thus, the charged amino acids in the first and/or second interaction region are directly connected.

In a preferred embodiment, the first interaction region comprises four consecutive arginines. According to one embodiment of the first aspect of the invention, the majority of the acidic amino acids are glutamic acids. According to an alternative embodiment, the majority of acidic amino acids are aspartic acids. Preferably, all acidic amino acids in the second peptide are glutamic acids. In particular the first interaction region comprises the sequence EEEE and the second interaction region comprises the sequence RRRR.

According to one embodiment the first interaction comprises the sequence EGEGEGE and the second interaction region comprises the sequence RGRGRGR. According to the one embodiment the first interaction comprises the sequence ESESESE and the second interaction region comprises the sequence RSRSRSR.

The spacing region between the covalent interaction pair or pairs and the non-covalent interaction pair or pairs has an influence on the formation of the hair pin-like structure of the second peptide. If the number of amino acids forming the spacer is too high, the effect of bringing the amino acids of the covalent interaction pair proximity by means of the binding of the one or more non-covalent interaction pairs may be lost. In contrast, a too short distance may be problematic for steric reasons. For example, the size of the side chains of the acidic and basic amino acids is bigger than the size of the side chain of cysteines. Accordingly, if the cysteines are directly adjacent to the charged amino acids in the second peptide a disulfide bridge might not form. Thus, according to one embodiment of the first aspect of the invention, the number of amino acids in the first and second interaction region between the at least one covalent interaction pair and the closest non-covalent interaction pair is in the range from 1 to 6, preferably 1 to 4, more preferably 1 to 3. Most preferably, the spacers in both interaction regions between the at least one covalent interaction pair and the closest non-covalent interaction pair is 2 amino acids.

In addition, the type of amino acids forming the spacer between the at least one covalent interaction pair and the closest non-covalent interaction pair influences the formation of the covalent bond. For the spacers, polar uncharged amino acids, with short side chains are preferred such as glycine, serine or alanine. More preferably the amino acids of the spacers between the at least one covalent interaction pair and the closest non-covalent interaction pair are glycine and serine. According to a particularly preferred embodiment of the invention, the spacers between the covalent interaction pair and the non-covalent interaction pair consist of one glycine and one serine. According to one embodiment of the fusion protein the first interaction region is Lyp-1 interaction region 1 as defined by SEQ ID NO: 20. According to one embodiment of the fusion protein the second interaction region is Lyp-1 interaction region 2 as defined by SEQ ID NO: 21. According to one embodiment the amino acid sequence of the first interaction region is defined as Lyp-1 interaction region 1 and the amino acid sequence of the second interaction region is defined as Lyp-1 interaction region 2.

Preferably, the second peptide is introduced into a region of the first peptide that is not essential for folding so that the second peptide does not interfere with the folding of the first peptide. Moreover, it is preferred that the second peptide is introduced into a region of the first peptide that is located on the surface of the first peptide when folded. The skilled person knows how to determine suitable positions within an amino acid sequence. Suitable positions are preferably determined from crystal structures of the protein or related proteins. Preferably, the second peptide is located in a loop of the first peptide of the fusion protein. More preferably in a loop on the surface of the first peptide.

The second peptide is particularly useful as a targeting peptide for targeting virus-like particles (VLPs). VLPs may comprise cargo that is useful only in specific cell types or toxic and must therefore address only specific cell types. Accordingly, it is preferred that the first peptide is a protein forming the capsid of a VLP.

According to a preferred embodiment, the first peptide is a polyoma virus VP1. "VP1" or "virus protein 1" according to the invention refers to a protein which is identical to or derived from the natural VP1 of the JC virus, having the amino acid sequence according to SEQ ID NO: 12. A protein derived from the natural VP1 of the JC virus preferably has an amino acid sequence homology or identity with the amino acid sequence according to SEQ ID NO: 12 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP1. The terms "VP1 derived from the natural VP1 of the JC virus" and "VP1 derived from JC virus" in particular also include VP1, which is identical to the natural VP1 of the JC virus. The term "VP1" according to the invention also encompasses fractions and derivatives of the natural VP1, which are capable of assembling into VLP. Preferably, said fractions and derivatives of VP1 at least comprise amino acids 32 to 316 of the amino acid sequence according to SEQ ID NO: 9 or a derivative thereof. Having a homology or identity with the amino acid sequence from amino acid position 32:316 of SEQ ID NO: 9 of at least 80 %, of at last 85 %, of at least 90 %, of at least 95 %, of at least 97 %, of at least 98 %, or of at least 99 %.

Preferably, the first peptide is a VP1 from JCV. According to X-ray crystallography analysis the folded VP1 (e.g. PDB entry 3NXD) contains three loops on the surface of the protein. Two of these loops, the DE-loop (aa 120-137) and the HI-loop (aa 262-272) are known to be eligible for the introduction of exogenous peptide structures as an exogenous structure introduced into the loop is accessible and in general does not interfere with folding of the VP1 protein. Thus, according to one embodiment, the second peptide is located in the DE-loop or the HI-loop of VP1. Preferably, the peptide is located between amino acid 120 and 137 (DE-loop) or 262 and 272 (HI-loop) of VP1. More preferably between amino acid 129 and 132 (DE-loop) or 265 and 268 (HI-loop) of VP1. As described below, different expression systems are suitable for the expression of the protein. According to a preferred embodiment, the fusion protein is expressed in *E.coli.*

According to one embodiment of the fusion protein the first peptide is a VP1 from JCV and the second peptide comprises the Lyp-1 peptide. According to one embodiment the first peptide is a VP1 from JCV and the second peptide comprises the amino acid sequence with a sequence homology or identity with the amino acid sequence according to SEQ ID NO: 13 of at least 95 %. According to one embodiment the first peptide is a VP1 from JCV and the second peptide comprises a amino acid sequence according to SEQ ID NO: 13. According to one embodiment the first peptide is a VP1 from JCV, the second peptide comprises a amino acid sequence according to SEQ ID NO: 13 and the second peptide is integrated into the DE-loop of the VP1. According to one embodiment the first peptide is a VP1 from JCV, the second peptide comprises an amino acid sequence according to SEQ ID NO: 13 and the second peptide is integrated into the HI-loop of the VP1. The fusion protein may have an amino acid sequence with a sequence homology or identity with the amino acid sequence according to SEQ ID NO: 14 of at least 95 %. Alternatively, the fusion protein may have an amino acid sequence with a sequence homology or identity with the amino acid sequence according to SEQ ID NO: 15 of at least 95 %.

### 3. Virus-Like Particle

According to a second aspect, the invention provides a virus-like particle (VLP) which comprises at least one fusion protein according to a first aspect of the invention. Preferably, the VLP is a polyoma virus VLP and the first peptide of the fusion protein is a polyoma virus VP1.

Non-limiting examples for viruses of the polyoma family are: B-lymphotropic polyomavirus (formerly known as African green monkey polyomavirus, AGMPyV) (LPyV), Baboon polyomavirus 1 (SA12), Bat polyomavirus (formerly known as Myotis polyomavirus, MyPyV; BatPyV) BK polyomavirus (BKPyV), Bornean orang-utan polyomavirus (OraPyV1), Bovine polyomavirus (BPyV), California sea lion polyomavirus (SLPyV), Hamster polyomavirus (HaPyV), JC polyomavirus (JCPyV), Merkel Cell polyomavirus (MCPyV), Murine pneumotropic virus (formerly known as Kilham strain of polyomavirus, Kilham virus, K virus; MPtV), Murine polyomavirus (MPyV), Simian virus 40 (formerly known as Simian vacuolating virus 40; SV40), Squirrel monkey polyomavirus (SqPyV), Sumatran orang-utan polyomavirus (OraPyV2), Trichodysplasia spinuolsa-associated polyomavirus (TSPyV), Human polyomavirus 6 (HPyV6), Human polyomavirus 7 (HPyV7), KI polyomavirus (formerly known as Karolinska Institute polyomavirus, KIPyV), WU polyomavirus (formerly known as Washington University polyomavirus, (WUPyV), Avian polyomavirus (formerly known as Budgerigar Fledgling disease polyomavirus, BFPyV, APyV), Canary polyomavirus (CaPyV), Crow polyomavirus (CPyV), Finch polyomavirus (FPyV), Goose Hemorrhagic polyomavirus (GHPyV), Athymic rat polyomavirus (RatPyV), Baboon polyomavirus 2 (BPyV2), Cynomolgus polyomavirus (CyPV), Gorilla gorilla gorilla polyomavirus 1 (GggPyV1), Human polyomavirus 9 (HPyV9), Trichodysplasia spinulosa-associated polyomavirus (TSV)Mastomys polyomavirus (multimammate mouse - Mastomys species), Pan troglodytes verus polyomavirus 1a (PtvPyV1a), Pan troglodytes verus polyomavirus 2c (PtvPyV2c), Rabbit kidney vacuolating virus (RKV).

Preferably the VLP is derived from a human polyoma virus comprising Human polyomavirus 6 (HPyV6), Human polyomavirus 7 (HPyV7), Human polyomavirus 9 (HPyV9), BK polyomavirus (BKPyV), JC polyomavirus (JCPyV), Merkel Cell polyomavirus (MCPyV), KI polyomavirus (formerly known as Karolinska Institute polyomavirus, KIPyV), WU polyomavirus (formerly known as Washington University polyomavirus, (WUPyV), Trichodysplasia spinulosa-associated polyomavirus (TSV), human polyoma virus 10 (HPyV10), MW polyomavirus and MX polyomavirus. In a more preferred embodiment of the invention, the VLP is derived from the human polyoma virus JCV.

VLPs are multi-protein structures that mimic the organization and conformation of authentic native viruses but lack the viral genome.

The virus-like particle according to the invention is preferably derived from human polyomavirus. In the context of the invention, the term "from human polyomavirus" refers to a VLP with structural proteins that can be isolated or extracted from polyomaviruses or which can be generated by recombinant expression of a polyoma structural protein or a modified form of said structural protein.

The capsids of all polyomaviruses have a similar structural set-up including the proteins VP1, VP2, VP3, and agnoprotein. The icosahedral virus capsid is formed by 72 VP1 pentamers. In the center of each of the pentamers, facing to the inside of the capsid, a VP2 or VP3 protein is located. VP3 is identical to the C-terminal two-thirds of VP2. This shared region comprises *inter alia* the nuclear localization signal (NLS), the DNA-binding domain (DBD), and the VP1 interacting domain (VID).

"VP2" or "virus protein 2" according to the invention refers to a protein which is identical to or derived from the natural VP2 of the JC virus, having the amino acid sequence according to SEQ ID NO: 22. A protein derived from the natural VP2 of the JC virus preferably has an amino acid sequence homology or identity with the amino acid sequence according to SEQ ID NO: 22 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP2.

"VP3" or "virus protein 3" according to the invention refers to a protein which is identical to or derived from the natural VP3 of the JC virus, having the amino acid sequence according to SEQ ID NO: 23. A protein derived from the natural VP3 of the JC virus preferably has an amino acid sequence homology or identity with the amino acid sequence according to SEQ ID NO: 23 of at least 80 %, of at least 85 %, of at least 90 %, of at least 95 %, of at least 97 %, of at least 98 %, or of at least 99 %, or with a sequence of at least 100 contiguous amino acids, preferably of at least 150, of at least 200, of at least 250, of at least 300 contiguous amino acids. Most preferably, the amino acid sequence homology or identity is calculated over the entire length of the natural JCV-VP3.

With the integration of a fusion protein according to the first aspect of the invention, the VLP may be targeted to a cell of interest. Polyomavirus VLPs are formed up to 72 pentamers. Each of these pentamers consists of 5 copies of VP1. Preferably, at least one pentamer of the icosahedral VLP capsid is formed by 1 to 5 copies of the fusion protein of the first aspect of the invention. Pentamers of the VLP may comprise 1, 2, 3, 4 or 5 copies of the fusion protein according to the first aspect. As the pentamers form upon expression and are stable also upon disassembly of the VLP, recombinant expression of only the fusion protein in a host cell generally leads to the formation of pentamers with 5 copies of the fusion protein.

According to one embodiment of the second aspect of the invention the ratio of pentamers formed by the fusion protein and pentamers formed by other VP1 is at least 1:71, at least 1:35, at least 1:23, at least 1:17, at least 1:11, at least 1:8, at least 1:7, at least 1:5, at least 1:3 at least 1:2. In this regard other VP1 proteins are VP1 constructs that do not include a fusion protein according to the invention. Preferably the ratio is at least 1:5. The higher the number of the fusion protein according to the invention in a VLP the higher the chance that the VLP will interact with a cell type specific for the targeting region of the fusion protein. However, the integration of a peptide into the structure of the VP1 may interfere with assembly of the VLP. Thus, it is preferred that at least a fraction of the pentamers is formed by VP1 without a peptide insertion, preferably by a VP1 with a sequence identity of at least 90 %, of at least 95 %, of at least 97 %, of at least 98 %, or of at least 99 % to SEQ ID NO: 9.

The VLP according to the second aspect of the invention may comprise more than one type fusion protein according to the first aspect of the invention. For example, the VLP may comprise one type of fusion protein with a targeting region specific for a first target and a second type of fusion protein with a targeting region specific for a second target. Such a VLP is able to target to different targets. The two targets may for example be epitopes located on different cell types. Accordingly, at least two different types of cells can be targeted. Alternatively, the two targets may be located on one type of cells. In this scenario the two targeting regions increase the chance of affecting the specific cell type by the VLP. The VLP with more than one fusion protein according to the first aspect of the invention may comprise a first fusion protein with the Lyp1-peptide as targeting region and a second fusion protein with the LTVSPWY-peptide as targeting region. The VLP with more than one fusion protein according to the first aspect of the invention may further comprise a first fusion protein with the Lyp1-peptide as targeting region and a second fusion protein with the F3-peptide as targeting region. This VLP is particularly useful for targeting breast tumor cells. Moreover, the VLP with more than one fusion protein according to the first aspect of the invention may comprise a first fusion protein with the SP5-2-peptide as targeting region and a second fusion protein with the F3-peptide as targeting region.

The VLP according to the second aspect may further comprise minor capsid proteins such as VP2, VP3 or agno protein. The VLP preferably comprises a fusion protein comprising a VP1 binding protein, which preferably comprises the VP1 interacting domain of VP2 and an exogenous peptide, preferably selected from a cargo binding peptide (CBP) and endosomal translocating peptide (ETP).

According to one embodiment of the second aspect, the VLP comprises a second fusion protein comprising a VP1 binding protein and an exogenous peptide, wherein the exogenous peptide comprises a cargo-loading peptide and/or an endosomal translocating peptide (ETP).

The term "VP1 binding protein" refers to any peptide that has the ability to bind to the major capsid protein VP1 of a polyomavirus. In particular, the VP1 binding protein is a peptide comprising the VP1 interacting domain (VID) of a polyomavirus VP2/VP3 protein.

The VID may be derived from a VP2 or VP3 or differently termed functional equivalent thereof from any known polyomavirus.

The VP1 binding protein according to the invention comprises the VP1 interacting domain of VP2 and allows a positioning of the fusion protein and, in particular, the exogenous peptide within a VLP derived from a polyomavirus. Preferably, the VP1 interacting domain has an identity of at least 90 %, preferably at least 95 %, more preferably at least 98 % to SEQ ID NO: 24. Thus, the VP1 binding protein preferably comprises at least a sequence with an identity of at least 90 %, preferably at least 95 %, more preferably at least 98 % to SEQ ID NO: 24.

The VP1 binding protein is preferably a full length polyomavirus VP2 or VP3. These proteins are naturally adapted for the interaction with the VP1. Accordingly, in one embodiment of the first aspect of the invention, the VP1 binding protein comprises an amino acid sequence that has an identity of at least 80 %, preferably at least 90 %, more preferably at least 95 % to SEQ ID NO: 22 or SEQ ID NO: 23.

However, any fragment or sub-structure of VP2 or VP3 may be sufficient for a tight interaction with VP1 as long as it contains a functional VP1 interacting domain of VP2/VP3. For example, the VP1 binding protein according to the invention may include or exclude the DNA-binding domain. For example, the VP1 binding protein may comprise the VID and the NLS of VP2. Further examples are a VP1 binding protein comprising the VID and the NLS of VP2, a VP1 binding protein comprising the VID and the DBD of VP2, and VP1 binding protein comprising the VID, DBD and the NLS of VP2.

The VP1 binding protein may be a modified version of VP2 or VP3, e.g. mutated by insertion, deletion, or amino-acid replacement with respect to SEQ ID NO: 22 or 23. However, the VP1 binding protein may only be modified to the point that the VP1 interacting domain is still functional, i.e. still binds to a polyomavirus VP1.

The exogenous peptide may be located at any position of the second fusion protein, i.e. at the C-terminus, at the N-terminus, or at any position within the amino acid sequence of the fusion protein. The location of the exogenous peptide is preferably on the surface of the folded protein. The exogenous peptide is further preferably freely accessible when the second fusion protein is bound to the VLP capsid. The skilled person knows how to determine positions within the amino acid sequences that fulfill these prerequisites. The structure predictions of VP2 or VP3 show that the N-terminus and the C-terminus are located on the surface of VP2 and VP3, and oriented to the inside of the polyoma virus when VP2 or VP3 is bound to a VP1 pentamer.

In one embodiment of the second aspect of the invention the exogenous peptide forms the C-terminus or the N-terminus of the second fusion protein. A second fusion protein containing the exogenous peptide on the C-terminus or N-terminus of the protein has the further advantage of an easier construction of the polynucleotide encoding the fusion protein. The C-terminus is particularly preferred as the location for the exogenous peptide because it is the part of the protein that is the last to be translated. Thus, an exogenous peptide on the C-terminus has the lowest influence on protein folding. According to one embodiment of the first aspect, the endosomal translocating peptide, preferably the CPP, is located on the C-terminus of the protein. Alternatively, the endosomal translocating peptide, preferably the CPP, forms the N-terminus of the second fusion protein. According to an alternative embodiment, the cargo loading peptide, in particular the cargo binding peptide, forms the C-terminus of the second fusion protein. Alternatively, the cargo binding peptide may form the C-terminus.

The exogenous peptide preferably has a percentage of basic amino acids of at least 25 %, more preferably of at least 30 %.

According to one embodiment of the first aspect of the invention, the exogenous peptide comprises a cargo loading peptide. A cargo loading peptide according to the invention is a peptide that affects the packaging of cargo in a VLP such that the cargo is better protected from the surrounding of the VLP in particular in the blood plasma or inside a cell.

Preferably the cargo loading peptide is a cargo-binding peptide. Depending on the application of the VLP it may be used for transporting different types of cargo. Examples of cargo are single- or double-stranded DNA, single- or double-stranded RNA, peptides, hormones, lipids, carbohydrates, or other small organic compounds. Accordingly, the cargo-binding peptide may be specific for one or more of these possible cargos. A preferred cargo-binding peptide is a DNA-binding peptide. A further preferred cargo-binding peptide is an RNA-binding peptide.

As shown in the examples, a cargo-loading peptide fused to the VP2 or VP3 protein leads to an improved protection, i.e. less degradation, of DNA packaged into VLPs. Without being bound to theory, one explanation for this better protection of the DNA cargo is a tighter packaging of the VLP due to the improved interaction with the cargo. Accordingly, the cargo-loading peptide is in particular a cargo binding peptide. Wildtype polyomavirus VP2/VP3 already contain a DNA-binding domain located at the C-terminus. However, the addition of protamine-1 to the C-terminus VP2 or VP3 leads to an improved protection of the cargo with respect to the wild type VP2 or VP3.

The length of the cargo-binding peptide is in principle only limited by the requirement that it does not interfere with the folding of the fusion protein. However, the cargo-binding peptide preferably has a length in the range from 5 to 100 amino acids, more preferably in the range from 10 to 70 amino acids, most preferably in the range from 10 to 60 amino acids. In one embodiment, the length is in the range from 15 to 25 amino acids.

According to one embodiment of the second aspect of the invention, the amino acid sequence of the cargo-binding peptide has a percentage of basic amino acids of at least 40 %. Basic amino acids are positively charged. The positive charge facilitates a binding to negatively charged cargo, e.g. nucleotides. Preferably, the majority of the basic amino acids of the cargo-binding peptide are arginine residues. Accordingly, the percentage of arginine residues in the sequence of the cargo-binding peptide is at least 20 %, more preferably at least 25 %, most preferably at least 35 %. In a particularly preferred embodiment, the percentage of arginine is at least 40 %.

According to one embodiment of the cargo-binding peptide comprises a structural motif (R)ₙ wherein n is an integer of at least 2, at least 3, at least 4, at least 5.

Cargo-binding peptides according to the invention may be DNA-binding peptides, RNA-binding peptides, peptide-binding peptides, lipid-binding peptides, carbohydrate-binding peptides. Examples of such peptides are protamine-1 (PRM1), Snap tag, SAMp73 Preferably, the CBP in the fusion protein according to the invention is protamine-1. Besides cargo-binding peptides the group of cargo-loading peptides also includes for example GFP or EGFP. According to one embodiment of the fusion protein according to the first aspect of the invention the cargo-loading peptide is neither GFP nor EGFP.

According to a further embodiment of the second aspect of the invention the amino acid sequence of the cargo-loading peptide has an identity of at least 80 %, preferably of at least 90 %, more preferably of at least 95 % to SEQ ID NO: 25 (Protamine-1) or SEQ ID NO: 26(Protamine-1aa8-29). A sequence identity to SEQ ID NO: 25 is particularly preferred. As shown in the examples, protamine-1 bound to either VP2 or VP3 has a strong effect on the protection of the cargo transported by VLPs.

According to an alternative embodiment of the second aspect of the invention the exogenous peptide comprises an endosomal translocating peptide (ETP).

An "ETP" according to the invention is a peptide that has the ability to translocate itself and any cargo bound to it through the endosomal membrane. Preferred endosomal translocating peptides are in particular cell-penetrating peptides (CPP). Cell-penetrating peptides (CPPs) are short peptides that facilitate cellular uptake of various molecular cargo (from nano-size particles to small chemical molecules or large fragments of DNA). The cargo is associated with the peptides either through chemical linkage via covalent bonds or non-covalent interactions. The functions of the CPPs are to deliver the cargo into the cells. A process that commonly occurs through endocytosis with the cargo delivered to the endosomes of the living mammalian cells. However, other peptides not classified as CPPs have the same function providing means to translocate through the endosomal membrane. Such peptides are also included in the definition of ETPs. One example for such a peptide is a polyhistidine peptide. A polyhistidine peptide consists of at least six histidine (His) residues. It was shown that a polyhistidine peptide also has a destabilizing effect on membranes. According to one embodiment of the first aspect of the invention the ETP is not a His₆-tag.

CPPs typically have an amino acid composition that either contains a high relative abundance of positively charged amino acids such as lysine or arginine, or has sequences that contain alternating pattern of charged amino acids and non-polar/hydrophobic amino acids. These two types of structures are referred to as polycationic or amphiphatic, respectively. A third class of CPPs are hydrophobic peptides, containing only apolar residues with a low net charge or have hydrophobic amino acid groups that are crucial for cellular uptake.

The mechanism by which the CPPs translocate the plasma membrane and facility the delivery of molecular cargo to the cytoplasm or an organelle is not entirely understood. However, the theories of CPP translocation can be classified into three main entry mechanisms: direct penetration in the membrane, endocytosis-mediated entry, and translocation through the formation of a transitory structure.

The CPP is in theory not limited in length; however, the peptide must allow a correct folding of the fusion protein. The cargo-binding peptide preferably has a length in the range of 5 to 100 amino acids, more preferably in the range from 10 to 30 amino acids, most preferably in the range from 15 to 25 amino acids.

Preferably, the CPPs contain in addition to the basic amino acids also non-polar amino acids. In particular, the CPP has a percentage of non-polar amino acids of at least 25 %, preferably of at least 30 %, more preferably of at least 35 %. The groups of CPPs differ in their relative percentage of basic non-polar amino acids.

The first type of CPPs, the amphipathic CPPs consist of alternating basic and non-polar amino acids. The amphipathic form often generates a pore or channel through the membrane bilayer. Examples of amphipathic CPPs are the trans-activating transcriptional activator (TAT) from human immunodeficiency virus-1 (HIV-1) and penetratin, a peptide derived from the DNA-binding domain of antennapedia homeo protein. The second type of CPPs, the so-called polycationic CPPs include the HPV peptide L2. These CPPs comprise at least one cluster of basic amino acids adjacent to at least one cluster of hydrophobic amino acids. Both regions are required for full activity of the peptide. Without being bound to theory, scientific results suggest that the positive charge of the basic amino acid cluster mediates tight association with negatively charged lipids of the membranes and that subsequent insertion of the hydrophobic cluster into membranes induces a torsional stress which results in membrane disruption.

Amphipathic CPPs in particular have a percentage of basic amino acids in the range from 40 to 60 %, and a percentage of non-polar amino acids in the range from 28 to 39 %. The amphipathic CPPs preferably have a percentage of arginines in the range from 18 to 36 %, and a percentage of lysines in the range from 22 to 28 %.

The polycationic CPPs preferably have a percentage of arginines in the range from 26 to 30 %, and a percentage of lysines in the range from 3 to 8 %.

According to one embodiment of the first aspect of the invention, the amino acid sequence of the CPP comprises a structural motif (R)ₙ, wherein ₙ is an integer of at least two, preferably of at least three, more preferably of at least four, and the sequence further comprises two or more adjacent non-polar amino acids. Polycationic CPP, such as HPV 33-L2, may have a sequence of four arginines and a sequence of three non-polar amino acids.

Preferred CPPs according to the invention are TAT, penetratin, and HPV 33-L2. TAT has an amino acid sequence as defined by SEQ ID NO: 27. Penetratin has an amino acid sequence as defined by SEQ ID NO: 28, and HPV 33-L2 has an amino acid sequence as defined by SEQ ID NO: 29. A further preferred CPP is a variant of HPV 33-L2, which is identified as HPV 33-L2-DD447 (SEQ ID NO: 30), differs from HPV 33-L2 by a replacement of the N-terminal phenylalanine and isoleucine by two aspartates. This variant was shown to have a stronger cell-penetrating effect (Kemper et al., 2006). Further examples of CPPs according to the invention are SynB1 (SEQ ID NO: 31), SynB3 (SEQ ID NO: 32), PTD-4 (SEQ ID NO: 33), PTD-5 (SEQ ID NO: 34), FHV Coat-(35-49) (SEQ ID NO: 35), BMV Gag-(7-25) (SEQ ID NO: 36), HTLV-II Rex-(4-16) (SEQ ID NO: 37), D-Tat (SEQ ID NO: 38), R9-Tat (SEQ ID NO: 39).

Thus, according to one embodiment of the first aspect of the invention, the amino acid sequence of the CPP has an identity of at least 80 %, preferably of at least 90 %, more preferably of at least 95 %, most preferably of at least 98 % to SEQ ID NO: 27. Alternatively, the amino acid sequence of the CPP has an identity of at least 80 %, preferably of at least 90 %, more preferably of at least 95 %, most preferably of at least 98 % to SEQ ID NO: 28. The sequence of the CPP may also have an identity of at least 80 %, preferably of at least 90 %, more preferably of at least 95 %, most preferably of at least 98 % to SEQ ID NO: 29. Moreover, the amino acid sequence of the CPP may have an identity of at least 80 %, preferably of at least 90 %, more preferably of at least 95 %, most preferably of at least 98 % to SEQ ID NO: 30.

According to one embodiment of the first aspect of the invention, the fusion protein comprises at least one exogenous cargo-binding peptide, and at least one exogenous CPP. That way, the fusion protein may provide to a VLP, which is used as a transport system for a specific cargo into a cell, a tighter packaging, an improved protection of the cargo, and, in addition, an improved ability to leave the endosomal pathway and arrive at the cytoplasm. Consequently, the combination of an ETP, in particular a CPP, and a cargo-loading peptide, in particular a cargo-binding peptide together in one fusion protein with the ability to bind to the major structural protein VP-1 strongly increases the yield of cargo entering the cytoplasm of a cell and thus increases the efficiency of a VLP mediated transport into a cell.

In a fusion protein comprising both a cargo-binding peptide and a CPP, the two peptides may be located at opposite termini of the fusion protein. Thus, either the cargo-binding peptide forms the N-terminus of the fusion protein, and the CPP forms the C-terminus of the fusion protein, or the cargo-binding peptide forms the C-terminus of the fusion protein and the CPP forms the N-terminus of the fusion protein. Both termini of VP2 and VP3 are presented to the surface of the protein and to the inside of the VLP when the VP2/VP3 is attached to VP1. VP2 and VP3 both contain a DNA-binding sequence on the C-terminus of the peptide. Thus, preferably, the exogenous cargo-binding peptide forms the C-terminus of the fusion protein and the CPP forms the N-terminus of the fusion protein.

According to one embodiment of the second aspect of the invention, one exogenous peptide comprises the CPP and the cargo-binding peptide and forms the N- or C-terminus of the protein. A localization of the two peptides on the C-terminus is preferred. The localization on the C-terminus is advantageous in cases in which the exogenous peptide may interfere with the protein folding. As protein folding already occurs co-translationally, there will be less interference by an exogenous peptide bound to the C-terminus which is only translated in the end.

It is preferred that the first peptide of the fusion protein comprises VP1 from the same polyomavirus as the further VP1 proteins forming the VLP.

According to one embodiment of the second aspect of the invention, the VLP comprises no cargo. In the context of this invention, a VLP without cargo is also referred to as "empty VLP". Preferably, the empty VLP comprises a second fusion protein according to the invention wherein the VP1 binding protein is VP2.

Surprisingly, it was found that an empty VLP can be designed that exhibits cytotoxic properties when introduced into a cell. An example of a cytotoxic empty VLP is VLP comprising a second fusion protein according to the invention wherein the VP1 binding protein is VP2. Due to the cytotoxic effect this empty VLP may for example be used as chemotherapeutic agents. The cytotoxic effect is preferably cell specific. A high cell specificity of the cytotoxic effect has the advantage that only specific cell types can be targeted in order not to harm any healthy cells. Thus, an empty VLP preferably comprises targeting means for specific cells on which the cytotoxic effect should be acted on. This targeting means is provided by a fusion protein according to the first aspect.

According to an alternative embodiment of the second aspect of the invention, the VLP comprises a cargo. Non limiting examples of cargo are single-stranded or double-stranded DNA, single-stranded or double-stranded RNA, peptides, hormones, lipids, carbohydrates, or other small organic compounds or mixtures thereof. Preferably, the cargo is a substance that produces an effect in a eukaryotic cell, in particular a mammalian cell. A preferred type of cargo is a substance that is pharmaceutically active. Preferably, the cargo is a molecule able to act on RNA. More preferably, the cargo is siRNA.

According to one embodiment of the second aspect of the invention the VLP is for use as a medicament. In particular, the VLP is for use in the treatment of tumor diseases. In this regard the VLP provides a vehicle for the cargo, preferably a pharmaceutically active ingredient, to enter the cells of an organism. Thus, according to one embodiment the VLP is for use as a drug delivery system. For this purpose the VLP is loaded by a drug of interest. The loading of the drug is in particular performed by disassembly of the VLP into pentamers and reassembly in the presence of the cargo. The VLP drug delivery system is then used to deliver the loaded drug to a specific target.

In one embodiment according to the second aspect of the invention, the cargo is a pharmaceutically active substance that is not applicable by itself to a patient or leads to strong side effects. An example of a group of such substances is chemotherapeutic substances. According to a further embodiment, the cargo is a diagnostic agent. Preferably, the diagnostic agent is a substance used in imaging methods. More preferably, the diagnostic agent is a dye, in particular a fluorescent dye. Thus, according to one embodiment of the second aspect the VLP is used as a diagnostic method. Preferably the diagnostic method includes the visualization of metastases and/or tumors, in particular for the preparation of a surgery.

The treatment or diagnosis of a patient with a VLP according to the second aspect of the invention comprises the transfer of the cargo into a cell of an organism. Preferably, the organism is a mammal, more preferably, a human.

### 4. Pharmaceutical composition

According to a third aspect, the invention provides a pharmaceutical composition that comprises at least one VLP according to the second aspect of the invention, and at least one pharmaceutically acceptable excipient.

### 5. Polynucleotide

According to a fourth aspect, the invention provides an isolated polynucleotide that comprises a nucleic acid sequence encoding a fusion protein according to the first aspect of the invention.

The techniques used to isolate or clone a polynucleotide encoding a peptide are known in the art and include isolation from genomic DNA, preparation from cDNA, or a combination thereof. The cloning of the polynucleotides from such genomic DNA can be effected, e.g., by using the well-known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used.

The isolated polynucleotide preferably comprises a first part encoding first peptide and second part encoding the second peptide. The first part of the polynucleotide encoding the first peptide preferably has a degree of sequence identity to the JCV-VP1 coding sequence SEQ ID NO: 7 of at least 80 percent, at least 85 percent, at least 90 percent, at least 95 percent, at least 96 percent, at least 97 percent, at least 98 percent, at least 99 percent, or 100 percent. In one embodiment of the fourth aspect of the invention the first part of the polynucleotide encoding the VP1 binding protein hybridizes under very low stringency conditions, low stringency conditions, medium stringency conditions, medium-high stringency conditions, high stringency conditions, or very high stringency conditions with SEQ ID NO: 7.

According to one embodiment of the fourth aspect of the invention, the second part of the polynucleotide encoding the second peptide preferably has a degree of sequence identity to SEQ ID NO: 8 of at least 80 percent, at least 85 percent, at least 90 percent, at least 95 percent, at least 96 percent, at least 97 percent, at least 98 percent, at least 99 percent, or 100 percent, which encode a polypeptide having protease activity. Preferably, the second part of the polynucleotide encoding the second peptide preferably hybridizes under very low stringency conditions, low stringency conditions, medium stringency conditions, medium-high stringency conditions, high stringency conditions, or very high stringency conditions with SEQ ID NO: 8.

### 6. Expression vector

In a fifth aspect the invention also relates to expression vectors comprising a polynucleotide according to the fourth aspect of the invention. The expression vector further preferably comprises control elements such as a promoter, and transcriptional and translational stop signals. The polynucleotide of according to the fourth aspect and of the control elements may be joined together to produce a recombinant expression vector that may include one or more restriction sites to allow for insertion or substitution of the polynucleotide encoding the polypeptide at such sites. The polynucleotide may be inserted into an appropriate expression vector for expression. In creating the expression vector, the coding sequence is located in the expression vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide of the fourth aspect of the invention. The choice of the expression vector will typically depend on the compatibility of the expression vector with the host cell into which the expression vector is to be introduced. The expression vectors may be a linear or closed circular plasmid.

The expression vector may be adapted for cell-based or cell-free expression. The expression vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

Alternatively, the vector may be one that, when introduced into the host cell, is integrated into the genome and replicated together with the chromosome(s) into which it has been integrated. For integration into the host cell genome, the expression vector may rely on any other element of the expression vector for integration into the genome by homologous or non-homologous recombination. Alternatively, the vector may contain additional polynucleotides for directing integration by homologous recombination into the genome of the host cell at a precise location in the chromosome.

The vectors of the present invention preferably contain one or more (e.g., several) selectable markers that permit easy selection of transformed, transfected, transduced, or the like cells. A selectable marker is a gene the product of which provides for biocide or viral resistance, resistance to heavy metals, prototrophy to auxotrophs, and the like.

The procedures used to ligate the elements described above to construct the recombinant expression vectors of the present invention are well known to one skilled in the art (see, e.g., Sambrook et al., 1989, supra).

### 7. Host Cells

According to a sixth aspect the invention provides a host cell, comprising the expression vector according the fifth aspect of the invention. The expression vector according to the fifth aspect is introduced into a host cell so that the expression vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram positive bacterium or a Gram negative bacterium. Gram positive bacteria include, but not limited to, Bacillus, Streptococcus, Streptomyces, Staphylococcus, Enterococcus, Lactobacillus, Lactococcus, Clostridium, Geobacillus, and Oceanobacillus. Gram negative bacteria include, but not limited to, E. coli, Pseudomonas, Salmonella, Campylobacter, Helicobacter, Flavobacterium, Fusobacterium, Ilyobacter, Neisseria, and Ureaplasma. Preferably the host cell is E.coli.

The host cell may also be a eukaryote, such as a mammalian, insect, plant, or fungal cell. Preferably, the host cell is an insect cell, still more preferably a lepidopteran cell, and most preferably a cell selected from the group consisting of Sf9, Sf21, Express SF+, and BTITn-5B1-4 ("TN High Five").

### 8. Production Method

According to a seventh aspect the invention provides a process of producing the VLP according the second aspect of the invention.

The process at least comprises the steps of protein expression of the fusion protein according to the first aspect of the invention with a polynucleotide according to the fourth aspect of the invention as a template, purifying the fusion protein and assembling several copies of the fusion protein together with several copies of VP1 to form a VLP.

For expression of fusion protein cell-based or cell-free (*in vitro*) expression systems may be used. Common cell based systems are bacteria, such as *E.coli, B. subtilis,* yeast, such as *S.cerevisiae* or eukaryotic cell lines, such as baculovirus infected Sf9 cells mammalian cells like CHO or HeLa.

Cell-free *(In vitro)* protein expression is the production of recombinant proteins in solution using biomolecular translation machinery extracted from cells. Cell-free protein production can be accomplished with several kinds and species of cell extract. Extracts used for cell-free protein expression are made from systems known to support high level protein synthesis. For example cell-free extracts capable are made from *E. coli*, rabbit reticulocyte lysates (RRL), wheat germ extracts, or insects cell (such as SF9 or SF21) lysates.

Preferably, a cell based expression system is used. Thus, according to one embodiment of the sixth aspect of the invention the process of producing the VLP according the second aspect of the invention comprises at least the steps of:
a) introducing a polynucleotide according to the fourth aspect into a host cell;
b) culturing the transformed host cell in a medium under conditions leading to a protein expression with the nucleic acid as a template;
c) isolating the expression product from the cell; and
d) assembly of the expression product optionally with further viral proteins into the VLP.

Suitable host cells and expression vectors are described above. The use of baculo viruses together with insect cells, in particular SF9 cells, is preferred.

Methods of cultivation of host cells in a nutrient medium suitable for production of the fusion protein are well known in the art. For example, the host cell may be cultivated by shake flask cultivation, small-scale or large-scale fermentation (including continuous, batch, fed-batch, or solid state fermentations) in laboratory or industrial fermenters performed in a suitable medium and under conditions allowing the fusion protein to be expressed. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection).

Depending on the host/vector system used the fusion protein may or may not be secreted into the nutrient medium. In case it is secreted the polypeptide can be recovered directly from the medium. Otherwise the cells are separated from the culture medium and lysed.

Methods of cell lysis are known in the art. Non-limiting examples of the methods of cell lysis are mechanical disruption, liquid homogenization, sonication, freeze-thaw procedure or mortar and pestle.

The fusion protein may be recovered using methods known in the art. For example, the fusion protein may be recovered from the nutrient medium by conventional procedures including, but not limited to, centrifugation, filtration, extraction, spray-drying, evaporation, or precipitation.

The fusion protein may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides. Expressed VP1 assembles into VLPs upon expression. The VLP may be disassembled by incubation with a reducing agent and a chelating agent (e.g. 5 mM EDTA). A preferred reducing agent is DTT. The concentration of DTT is preferably in the range from 1 to 30 mM, more preferably the concentration of DTT is in the range from 5 to 15 mM, in particular about 1 mM. The chelating agent is preferably EDTA. The concentration of EDTA is preferably in the range from 1 to 15 mM, more preferably the concentration of is in the range from 2 to 10 mM, in particular about 5 mM. Suitable incubation conditions are for example a temperature in the range from 20 to 30 °C, preferably about 25°C a duration of 20 min to 2 h, preferably about 1 h and a shaking speed in the range from 200 to 2000 rpm preferably about 1000 rpm.

Under these conditions the VLP disassembles into VP1 pentamers. The VP1 pentamers can for example be reassembled into icosahedral VLPs by dialysis against 2 x 2 liter reassembly buffer (10 mM TrisHCl pH 7.4; 150 mM NaCl; 2 mM CaCl₂) for at least 84 h. In order to produce a VLP with the fusion protein the fusion protein may be incubated with the VP1 pentamers during the disassembly procedure (see above). The fusion protein and VP1 pentamers than reassemble during dialysis into mixed VLPs.

According to one embodiment of the process further comprises the steps:
f) disassembly of the VLP into pentamers;
g) mixture of the pentamers with wildtype VP1 pentamers; and
h) reassembly of VLPs from the pentamer mixture.

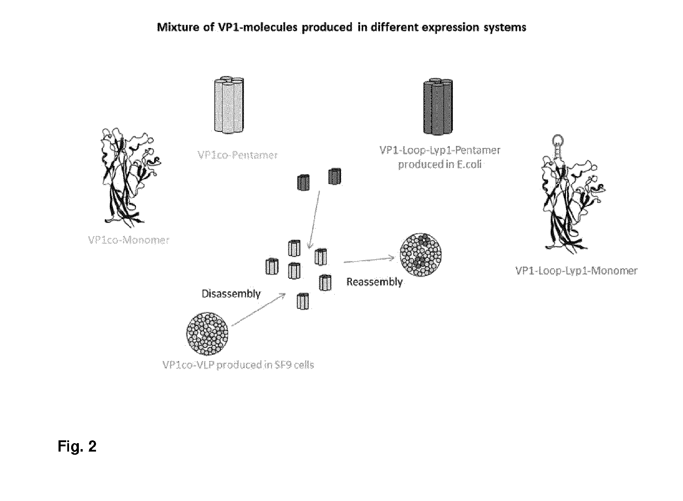

An overview of the process is shown in **Fig. 2****.**

The fusion protein according to the first aspect can for example be incorporated into the capsid envelope by co-expression of the respective fusion protein and VP1 in a suitable host cell e.g. a eukaryotic cell. In a preferred embodiment of the invention the fusion protein is co-expressed with a VP1 or a fusion protein comprising the VP1. In particular the fusion protein and the VP1 are coexpressed in Sf9 cells.

Active substances can be incorporated into the interior of the capsid envelope by for example dissociation of the capsid envelope and subsequent re-association in the presence of the active substance or by osmotic shock of the VLP in the presence of the active substance.

### EXAMPLES

### EXAMPLE 1: Cloning of VLP-variants with a cyclic Lyp-1-peptide in via whole vector PCR

### a) Introduction of the cyclic Lyp-1-peptide into the HI-loop

Primers:
Lvp-1peptid HI-Loop FW (SEQ ID NO: 40)
GAACTAGAGGGTGCGGATCCGAAGAGGAAGAGGGTTCCCAGCAGTGG
Lyp-1peptid HI-Loop RV (SEQ ID NO: 41)
GCTTGTTACCACAACCTGATCTACGTCTACGGTTGGTGAACATGC

Plasmid:
pTXB1_VP1 (SEQ ID NO: 42)

Before the PCR the primers were phosphorylated 20 min at 37°C and 10 min at 75°C in the following reaction set up:

| | |
|---|---|
| 50 pmol | primer |
| 2 µl | buffer A |
| 2 µl | 10 mM dATP |
| 10U | T4 polynucleotide kinase (Fermentas) |
| ad 20 µl | bidest H₂O. |

The following PCR mixture was prepared:

| | |
|---|---|
| 1.00 µl | pTXB1_VP1 (1 ng/µl) |
| 4.00 µl | 5x PCR Q5 Puffer (w 7.5 mM MgCl2) |
| 0.40 µl | 10 mM dNTP (200 µM) |
| 1.00 µl | Lyp-1 peptid_HI-Loop_FW (10µM) |
| 1.00 µl | Lyp-1 peptid_HI-Loop_RV (10µM) |
| 0.20 µl | Q5-Hot Start (2 U/µl NEB) (0.4 U) |
| 12.40 µl | H₂O MilliQ |

For PCR, the following temperature profile was used: An initial activation at 98 °C for 30 sec followed by 25 repetitions of the following cycle steps 1 to 3: 1) Denaturation: 98 °C for 10 seconds; 2) Annealing: 60 °C for 10 seconds; and 3) Extension: 72 °C for 3 min. After the temperature cycling, the samples were again kept at 72 °C for 10 min and finally cooled to 12 °C until the samples were retrieved.

PCR products (7771 bp) were separated by agarose gel electrophoresis and fragments of about 7400 bp were eluted using the QiaEx Kit (Qiagen).

Fragments were eluted with 30 µl of 70°C buffer 4 (NEB) and subsequently incubated with 2 µl Dpnl (digest of methylated template plasmid DNA) for 2 h at 37°C followed by an additional 20 min at 80°C for inactivation of the Dpnl. Afterwards the PCR fragments were religated to using the T4 ligase.

Ligation reaction set up:

| | |
|---|---|
| 2 µl | PCR-Product |
| 1 µl | 10x Ligase-buffer |
| 2.5 | Units Ligase |
| Ad 10 µl | ddH₂O |

For ligation the samples were incubated for 1 h RT.

The plasmids were then transformed into competent DH5α bacteria by thermal shock. Accordingly, 5 µl ligation sample were added to 50 µl competent XL1Blue cells and incubated for 30 min on ice, heated for 45 s at 42°C followed by further incubation for 2 min on ice. Afterwards, 500 µl SOC were added the cell culture was incubated for 30 min at 37°C. The cells were then harvested for 5 min at 3.000 x g, the cell pellets resuspended in 50 µl SOC and 20 µl of the cell suspension spread on "amp+" agar plates.

Single colonies growing on the amp+ agar plate were tested for correct insertion. For this, the plasmid DNA was prepared by DNA Miniprep (Qiagen) according to the manufacturers' protocol. The obtained DNA was digested with the restriction enzyme Agel. A construct with a correct insertion would yield two bands in agarose gel electrophoresis at 6703 base pairs and 1068 base pairs. Clones with correct restriction pattern were sequenced.

### b) Introduction of the cyclic Lyp-1-peptide into the DE-loop

For introduction of the Lyp-1-peptide into the DE-loop the protocol as described under a) was used with the following differences.

Primers:
Lyp-1peptid DE-Loop FW (SEQ ID NO: 43)
GAACTAGAGGGTGCGGATCCGAAGAGGAAGAGGGTGCTGGCAAGC
Lyp-1peptid DE-Loop RV (SEQ ID NO: 44)
GCTTGTTACCACAACCTGATCTACGTCTACGGTGGGTAGCCTGGC

The expected band size in the restriction analysis was 6760 base pairs and 1011 base pairs DE-Loop.

### EXAMPLE 2: Expression and Purification of VP1-Lyp1 fusion proteins in E.coli.

### a) Protein expression

### Expression vectors pTXB1_VP1-HI-Loop + Lyp1 and pTXB1_VP1-DE Loop + Lyp1 were produced according to Example 1.

For expression in *E.coli* the plasmids were transformed into the *E.coli* strain BL21 (DE3). For transformation, chemically competent cells were mixed with 1 ng of plasmids and incubated on ice for 30 min. Transformation was induced by heat shock for 45 s at 42°C. After this cells were transferred back to ice for at least 2 min before addition of SOC medium (37°C) and incubation for 1 h at 37°C under continuous shaking (225 rpm). For selection of positively transformed *E.coli BL21* the culture was spread out on LB agar plates containing ampicillin (LB-amp) as selective marker. For expression, a colony grown on the LB-amp plate was inoculated into 50 ml LB medium comprising ampicillin in a concentration of 100 µg/ml and grown overnight at 37°C and 225 rpm. This overnight culture was transferred into one liter LB-amp medium. Inoculated cultural medium was split into four 250 ml fractions and transferred into 1000 ml Erlenmeyer flasks. The four cultures were grown at a temperature of 37 °C with a shaking speed of 225 rpm until reaching an OD600 of 0.4. At this growth stage, protein expression was induced by an addition of 0.4 mM IPTG (end concentration). After induction of protein expression, the cells were grown for four hours at 30 °C with 225 rpm shaking.

### b) Protein preparation

Cells were harvested by centrifugation at 3,500 g and 4 °C for 15 minutes. The supernatant was discarded and the pellets were resuspended in 15 ml lysis buffer, joining two resuspended pellets in one 50 ml falcon tube.

Lysis Buffer
20 mM Tris-HCl, pH 8.5
500 mM NaCl
1 mM EDTA
1 mM TCEP
0.1% Triton-X 100

Lysozyme was added at a concentration of 20,000 units per milliliter of cell suspension. The cell suspension was then incubated for 30 minutes in an overhead shaker at 4 °C. After incubation into each of the falcons, 5 g of silica beads (0.1 - 0.2 mm, Mühlmeyer) were added. The cells were lysed in a BigPrep adapter with the FastPrep instrument (MPBio) at 6.5 m/s for 30 seconds in two repetitions. The lysed cells were then centrifuged at 10,000 g and 4 °C for 15 minutes. The supernatants were recovered and stored for further processing. The pellets were then resuspended in 15 ml Lysis Buffer and lysed using the FastPrep system as described above. The cells were again centrifuged at 10,000 g and 4 °C for 15 minutes. The supernatants were retrieved and joined with the stored supernatants.

### c) Protein purification

10 ml of chitin agarose were filled into a column. The column was washed with 100 ml of Column Buffer and stored at 4 °C.

Column Buffer
20 mM TrisHCL, pH 8.5
500 mM NaCl
1 mM EDTA

The supernatants of the protein preparation were again spun at 15,000 g at 4 °C for 30 minutes and afterwards applied onto the chitin column. The protein solution was allowed to pass the chitin column by gravity flow. After the protein solution has passed the column by gravity flow, the chitin column was washed with 200 ml column buffer by gravity flow. After the column buffer has run through, 30 ml cleavage buffer were added.

Cleavage Buffer
20 mM TrisHCl, pH 8.5
200 mM NaCl
1 mM EDTA
15 mM DTT

After the Cleavage Buffer has entered the gel, the chitin column was closed and incubated overnight at room temperature. After incubation, the cleavage buffer was removed by gravity flow. For elution, five 10 ml portions of elution buffer were added and let run through the column collecting the eluate.

Elution Buffer
20 mM TrisHCl, pH 8.5
200 mM NaCl
1 mM EDTA
2 mM DTT.

During the preparation and purification, samples were collected at the different steps and tested together with samples of the eluate using SDS-PAGE and Western

### d) Analysis of the protein production

SDS-PAGE was performed with two gels containing the same samples. One gel was stained with InstantBlue. The second one was used for Western Blot analysis. The proteins were transferred from the gel to a membrane for one hour at 1 mA/cm². Afterwards, free protein binding sites on the membrane were blocked for 30 minutes at room temperature in PBS-T containing 5 % (w/w) low fat milk powder. The low fat milk solution was removed and the first antibody AK 254 C7E4 diluted 1 to 5,000 in PBS-T plus 0,5 % (w/w) low fat milk powder was added to the membrane and incubated for one hour at room temperature. After incubation, the membrane was incubated three times for five minutes in PBS-T. The PBS-T was removed and the second antibody was applied. For this, a goat anti-mouse POX was diluted 1 to 10,000 in PBS-T + 0.5% low fat milk powder. The antibody solution was added to the membrane and incubated for one hour at room temperature. The membrane was again washed three times for 5 minutes in PBS-T. Bound secondary antibody was detected by chemoluminescence. Eluate fractions showing a positive signal were pooled and the volume was reduced by a factor of 10 using Viva spin 20 columns (molecular weight cut off 30,000). Protein content and purity of the final eluate were determined via an Experion analysis system (Biorad).

### EXAMPLE 3: Expression of VP1 in Sf9 and production of VLP

### a) Protein expression

Sf9 were grown to a cell density of 2 x 10⁷ in serum-free TC100 medium and infected with the recombinant baculovirus with a multiplicity of infection (MOI) of 5. After infection the cells were grown for 5 to 7 days at 27°C producing the VP1 protein encoded by the baculovirus. The produced protein is secreted into the expression medium. In the cell supernatant the secreted proteins self-assemble into VLPs.

### b) Purification of VLPs from the supernatant

Sf9 cells were separated from the supernatant by centrifugation for 5 min at 500 x g. Cells were discarded and the supernatant was centrifuged a second time for 90 min at 5000 x g in order to remove larger impurities.

The VLPs were then separated by ultracentrifugation. For this, 15 ml of clarified supernatant was loaded on 3 ml 40% sucrose.

Ultracentrifugation was performed in a Sorwall MX150 for 4h at 100000 x g and 4°C. In the centrifugation tube a pellet formed by the VLPs which was harvested. The VLP containing pellet was resuspended in Tris buffer (10 mM Tris, 150 mM NaCl, pH 7,5) The protein concentration of the resuspended VLPs was determined and adjusted to 0,5 µg/µl by the addition of Tris-Buffer.

### EXAMPLE 4: Production of VLPs containing a VP1-Lyp1 fusion protein

**Fig. 2** gives an overview of the production. In a first step, VP1 was produced as described in Example 3. VP1 with a Lyp-1 targeting peptide in the HI or DE loop were produced as described in Example 2.

The proteins were then mixed in a ratio of 5:1. Accordingly, 100 µl VP1 (500 ng/µl) were added to 200 µl VP1-HI-Loop-Lyp1 from *E.coli* (at a concentration of 50 ng/µl). The mixed VLPs were then disassembled by addition of 3 µl 0.5 M EDTA and 3 µl 1 M DTT at an incubation of 1 hour at 25 °C and 1,000 rpm shaking. The reassembly by dialysis against a calcium rich buffer then leads to the formation of mixed VP1 VLPs. The sample was dialysed for 60 hours against 2 litres of Reassembly Buffer.

### Reassembly Buffer

### PBS

1 mM CaCl₂
1 mM MgCl₂

After 60 hours, the Reassembly Buffer was replaced by another 2 litres of the same buffer and the sample was dialysed for another 24 hours. Afterwards, the protein concentration in the sample was determined using the PIERCE 660 nM protein assay. Samples were analysed using SDS-PAGE and Western Blot Analysis. According to the same protocol VLPs containing VP1-DE-Loop-Lyp1 were produced.

### EXAMPLE 5: Targeting test with VLPs containing a VP1-Lyp1 fusion protein

### a) Labelling of VP1 with fluorescent dyes

VP1 was produced using the established SF9/Baculo virus expression.

200 µl VP1 (0.5 mg/ml) from insect expression system were mixed with 10 µl of 0.2 M bicarbonate pH 9 and 0.5 µl 10 mM NHS-Ester of the fluorescent dye. The mixture was rotated at RT for 1 h before 1 µmol of ethanolamine was added to quench unreacted dye. Free dye was removed by dialysis against 2 x 1 Liter PBS containing 1 mM CaCl₂ and 1 mM MgCl₂ 24 h each.

### b) Production of VLPs with VP1-Lyp1 fusion proteins and dye labelled VP1

The following VLPs assembled by VP1-Lyp1 fusion proteins and dye labelled VP1 were produced according to the protocol in Example 3.
i) VLP with VP1-DE-Loop-Lyp1 and VP1-FITC
ii) VLP with VP1-HI-Loop-Lyp1 and VP1-FITC
iii) VLP with VP1-DE-Loop-Lyp1 and VP1-Atto488
iv) VLP with VP1-HI-Loop-Lyp1 and VP1-Atto488
v) VLP with VP1-DE-Loop-Lyp1 and VP1-Atto647
vi) VLP with VP1-HI-Loop-Lyp1 and VP1-Atto647

### c) Treatment of Cancer cells exhibiting p32 with the VLPs

The cancer cell line MDA-MB-231 (ATCC® HTB-26™) was applied into a 12-well plate with a cell number of about 200.000 cells per well. The cells are incubated at 37 °C and 5 % CO₂ in 2 ml of the culture medium DMEM. After 20 h of incubation 4 µg of a VLP construct as defined in i) to vi) was added in the culture medium and the cells were incubated for another 4 h at 37 °C and 5 % CO₂.

### d) Flow cytometry

After incubation with the VLP the culture medium was removed and the cells were washed by addition and subsequent removal of 2 ml of ice cold PBS. In the following cells were dissociated from the surface of the bottom of the well by addition of 500 µl T/E solution. To the cells 2 ml of PBS + 2 % (w/w) fetal calve serum (FCS) were added and the cell suspension were centrifuged for 10 min at 250 g and 5 °C. Afterwards the cells were washed three times with 2 ml of PBS + 2 % (w/w) FCS. The cell suspension were centrifuged for 10 min at 250 g and 4 °C and the resulting cell pellet suspended in 1 ml PBS with 1 µg/ml propidium iodide.

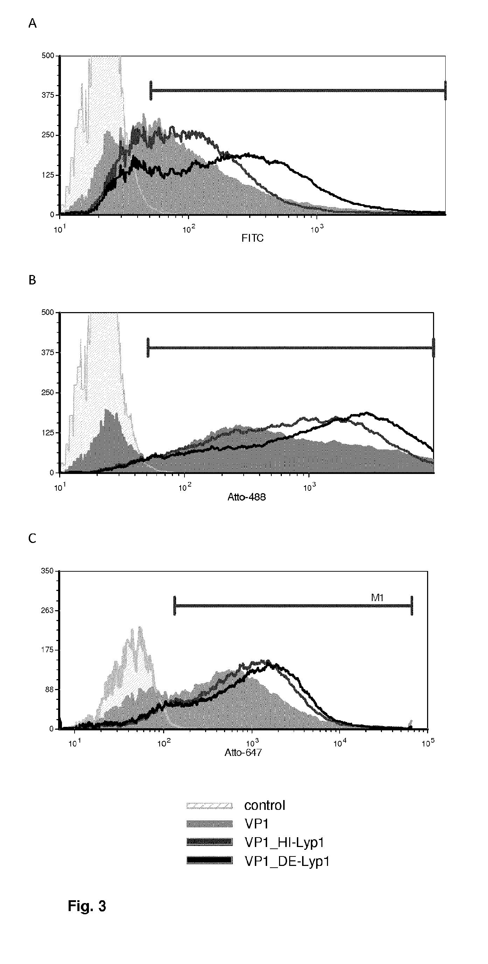

The results of the flow cytometry are shown in form of histograms in **Fig.** 3 **a) to c). Fig. 3** shows histograms calculated from flow cytometry data obtained from MDA-MB-231 cells treated with VLPs. The histogram curves in **Fig. 3 a)** **to c)** correspond to treatments with the VLPs from VP1, VP1-DE-Loop-Lyp1 or VP1-HI-Loop-Lyp1. In addition, a histogram from a control sample of untreated cells is present. In the histograms the X-axis represents the intensity of the measured signal, the Y-axis the number of cells with the specific signal intensity. In all experiments the histograms of the cell treatment with VLPs containing VP1-DE-Loop-Lyp1 or VP1-HI-Loop-Lyp1 are shifted to the right as compared to the cell treatment with VLPs containing only wildtype VP1. Thus, a larger number of cells take up the VLPs containing VP1 proteins with the targeting peptide (Lyo-1 as targeting region) according to the invention. An explanation for the increased uptake is the interaction of the Lyp-1 targeting region with the cancer cells exhibiting p32.

The results of the experiment are additionally shown in **Fig. 4** in form of a column diagram. In **Fig. 4** each column represents the percentage of cells with a positive uptake of VLPs, identified by minimum signal strength. **Fig. 4** shows that the number of cells with a positive uptake of VLPs is increased by about 20 % in the experiments with VLPs containing VP1-DE-Loop-Lyp1 and VP1-HI-Loop-Lyp1 compared to VLPs from normal VP1.

## Claims

1. A fusion protein comprising at least a first and a second peptide, wherein
- the second peptide comprises a targeting region and a first and a second interaction region,
- the second peptide is located on the surface of the fusion protein;
- the second peptide comprises at least two interaction pairs, wherein an interaction pair is formed by an amino acid of the first interaction region and an amino acid of the second interaction region,
- the interaction between the amino acids of an interaction pair is covalent or non-covalent; and
- at least one interaction pair is a covalent interaction pair in which the amino acids are covalently bound.

2. The fusion protein according to claim 1, wherein the first peptide is a polyoma virus VP1, preferably a VP1 from JCV.

3. The fusion protein according to claim 1 or 2, wherein the covalent interaction pair is formed by a cysteine in the first interaction region and a cysteine in the second interaction region.

4. The fusion protein according to any of the preceding claims, wherein at least two interaction pairs are non-covalent interaction pairs in which the amino acids interact non-covalently.

5. The fusion protein according to claim 4, wherein the 2 to 6 acid/base interaction pairs, preferably 3 to 5 non-covalent interaction pairs are acid/base interaction pairs.

6. The fusion protein according to claim 5, wherein the acid/base interaction pairs are formed by acidic amino acids in the first interaction region and basic amino acids in the second interaction region.

7. The fusion protein according to claim 6, wherein the majority of the basic amino acids are arginine, preferably all basic amino acids are arginines.

8. The fusion protein according to any of claim 6 or 7, wherein the majority of the acidic amino acids are glutamic acid, preferably all acidic amino acids are glutamic acids.

9. The fusion protein according to any of the preceding claims, wherein the number of amino acids in the first and/or second interaction region between the at least one covalent interaction pair the closest non-covalent interaction pair is in the range from 1 to 6 preferably 1 to 4, more preferably 2.

10. The fusion protein according to any of the preceding claims, wherein the amino acid sequence of the targeting region is selected from the group consisting of Lyp-1, RGD, RGR, HER-2 binding peptide, CREKA peptide, NGR peptide CPP-2, CPP-44, F3, RMS-P3, F56, LTVSPWY-peptide, and WNLPWYYSVSPT-peptide.

11. A virus-like particle (VLP) comprising at least one fusion protein according to any of claims 1 to 10.

12. The VLP according to claim 11, wherein the second peptide is located on the outer surface of the VLP capsid.

13. The VLP according to claim 11 or 12, further comprising a second fusion protein comprising a VLP binding protein and an exogenous peptide.

14. The VLP according to any of claims 11 to 13, wherein the VLP comprises no cargo.

15. The VLP according to any of claims 11 to 13, wherein the VLP comprises a cargo selected from single-stranded or double-stranded DNA or RNA, preferably siRNA, oligopeptides, polypeptides, hormones, lipids, carbohydrates, other small organic compounds or mixtures thereof.

16. The VLP according to any of claims 11 to 15 for use in a method of treatment or in a diagnostic method.

17. The VLP according to any of claims 11 to 16 for use as drug delivery system.

18. A pharmaceutical composition comprising the VLP according to any of claims 11 to 17 and at least one pharmaceutically acceptable carrier.

19. An isolated polynucleotide comprising a nucleic acid sequence encoding a fusion protein according to any claims 1 to 10.

20. An expression vector comprising the polynucleotide according to claim 19.

21. A host cell comprising the expression vector according to claim 20.

22. A process of producing the VLP according to any of claims 11 to 17, comprising the steps of:
a) introducing a polynucleotide according to claim 19 into a host cell;
b) culturing the transformed host cell in a medium under conditions leading to a protein expression with the nucleic acid as a template;
c) isolating the expression product from the cell; and
d) assembly of the expression product optionally with further viral proteins into the VLP.

23. The process according to claim 22, further comprising the steps:
f) disassembly of the VLP into pentamers;
g) mixture of the pentamers with wildtype VP1 pentamers; and
h) reassembly of VLPs from the pentamer mixture.


SEQUENCE LISTING

<110>  Life Science Inkubator Betriebs GmbH & Co. KG

<120>  VLP with a targeting peptide

<130>  55 321 K

<160>  44

<170>  PatentIn version 3.5

<210>  1
<211>  9
<212>  PRT
<213>  synthetic

<400>  1

Cys Gly Asn Lys Arg Thr Arg Gly Cys
1               5

<210>  2
<211>  6
<212>  PRT
<213>  synthetic

<400>  2

Lys Cys Cys Tyr Ser Leu
1               5

<210>  3
<211>  5
<212>  PRT
<213>  synthetic

<400>  3

Cys Arg Glu Lys Ala
1               5

<210>  4
<211>  15
<212>  PRT
<213>  Campylobacter spec.

<400>  4

Asp Ser Leu Lys Ser Tyr Trp Tyr Leu Gln Lys Phe Ser Trp Arg
1               5                   10                  15

<210>  5
<211>  15
<212>  PRT
<213>  Campylobacter spec.

<400>  5

Lys Arg Pro Thr Met Arg Phe Arg Tyr Thr Trp Asn Pro Met Lys
1               5                   10                  15

<210>  6
<211>  31
<212>  PRT
<213>  Homo sapiens (human)

<400>  6

Lys Asp Glu Pro Gln Arg Arg Ser Ala Arg Leu Ser Ala Lys Pro Ala
1               5                   10                  15

Pro Pro Lys Pro Glu Pro Lys Pro Lys Lys Ala Pro Ala Lys Lys
            20                  25                  30

<210>  7
<211>  12
<212>  PRT
<213>  synthetic

<400>  7

Cys Met Gly Thr Ile Asn Thr Arg Thr Lys Lys Cys
1               5                   10

<210>  8
<211>  12
<212>  PRT
<213>  synthetic

<400>  8

Trp His Ser Asp Met Glu Trp Trp Tyr Leu Leu Gly
1               5                   10

<210>  9
<211>  7
<212>  PRT
<213>  synthetic

<400>  9

Leu Thr Val Ser Pro Trp Tyr
1               5

<210>  10
<211>  12
<212>  PRT
<213>  synthetic

<400>  10

Trp Asn Leu Pro Trp Tyr Tyr Ser Val Ser Pro Thr
1               5                   10

<210>  11
<211>  16
<212>  PRT
<213>  synthetic

<400>  11

Asn Gly Tyr Glu Ile Glu Trp Tyr Ser Trp Val Thr His Gly Met Tyr
1               5                   10                  15


<210>  12
<211>  611
<212>  PRT
<213>  JC-Polyomavirus

<400>  12

Met Ala Pro Thr Lys Arg Lys Gly Glu Pro Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Asp Asn Gly Ala Gly Lys Pro Val Gln Gly Thr Ser Phe His Phe
    130                 135                 140

Phe Ser Val Gly Gly Glu Ala Leu Glu Leu Gln Gly Val Val Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys

            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Gly Ser Gln Gln Trp Arg Gly
            260                 265                 270

Leu Ser Arg Tyr Phe Lys Val Gln Leu Arg Lys Arg Arg Val Lys Asn
        275                 280                 285

Pro Tyr Pro Ile Ser Phe Leu Leu Thr Asp Leu Ile Asn Arg Arg Thr
    290                 295                 300

Pro Arg Val Asp Gly Gln Pro Met Tyr Gly Met Asp Ala Gln Val Glu
305                 310                 315                 320

Glu Val Arg Val Phe Glu Gly Thr Glu Glu Leu Pro Gly Asp Pro Asp
                325                 330                 335

Met Met Arg Tyr Val Asp Lys Tyr Gly Gln Leu Gln Thr Lys Met Cys
            340                 345                 350

Ile Thr Gly Asp Ala Leu Val Ala Leu Pro Glu Gly Glu Ser Val Arg
        355                 360                 365

Ile Ala Asp Ile Val Pro Gly Ala Arg Pro Asn Ser Asp Asn Ala Ile
    370                 375                 380

Asp Leu Lys Val Leu Asp Arg His Gly Asn Pro Val Leu Ala Asp Arg
385                 390                 395                 400

Leu Phe His Ser Gly Glu His Pro Val Tyr Thr Val Arg Thr Val Glu
                405                 410                 415

Gly Leu Arg Val Thr Gly Thr Ala Asn His Pro Leu Leu Cys Leu Val
            420                 425                 430

Asp Val Ala Gly Val Pro Thr Leu Leu Trp Lys Leu Ile Asp Glu Ile
        435                 440                 445

Lys Pro Gly Asp Tyr Ala Val Ile Gln Arg Ser Ala Phe Ser Val Asp
    450                 455                 460

Cys Ala Gly Phe Ala Arg Gly Lys Pro Glu Phe Ala Pro Thr Thr Tyr
465                 470                 475                 480

Thr Val Gly Val Pro Gly Leu Val Arg Phe Leu Glu Ala His His Arg
                485                 490                 495

Asp Pro Asp Ala Gln Ala Ile Ala Asp Glu Leu Thr Asp Gly Arg Phe
            500                 505                 510

Tyr Tyr Ala Lys Val Ala Ser Val Thr Asp Ala Gly Val Gln Pro Val
        515                 520                 525

Tyr Ser Leu Arg Val Asp Thr Ala Asp His Ala Phe Ile Thr Asn Gly
    530                 535                 540

Phe Val Ser His Ala Thr Gly Leu Thr Gly Leu Asn Ser Gly Leu Thr
545                 550                 555                 560

Thr Asn Pro Gly Val Ser Ala Trp Gln Val Asn Thr Ala Tyr Thr Ala
                565                 570                 575

Gly Gln Leu Val Thr Tyr Asn Gly Lys Thr Tyr Lys Cys Leu Gln Pro
            580                 585                 590

His Thr Ser Leu Ala Gly Trp Glu Pro Ser Asn Val Pro Ala Leu Trp
        595                 600                 605

Gln Leu Gln
    610

<210>  13
<211>  21
<212>  PRT
<213>  synthetic

<400>  13

Arg Arg Arg Arg Ser Gly Cys Gly Asn Lys Arg Thr Arg Gly Cys Gly
1               5                   10                  15

Ser Glu Glu Glu Glu
            20

<210>  14
<211>  632
<212>  PRT
<213>  synthetic

<400>  14

Met Ala Pro Thr Lys Arg Lys Gly Glu Pro Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Arg Arg Arg Arg Ser Gly Cys Gly Asn Lys Arg Thr Arg Gly Cys
    130                 135                 140

Gly Ser Glu Glu Glu Glu Gly Ala Gly Lys Pro Val Gln Gly Thr Ser
145                 150                 155                 160

Phe His Phe Phe Ser Val Gly Gly Glu Ala Leu Glu Leu Gln Gly Val
                165                 170                 175

Val Phe Asn Tyr Arg Thr Lys Tyr Pro Asp Gly Thr Ile Phe Pro Lys
            180                 185                 190

Asn Ala Thr Val Gln Ser Gln Val Met Asn Thr Glu His Lys Ala Tyr
        195                 200                 205

Leu Asp Lys Asn Lys Ala Tyr Pro Val Glu Cys Trp Val Pro Asp Pro
    210                 215                 220

Thr Arg Asn Glu Asn Thr Arg Tyr Phe Gly Thr Leu Thr Gly Gly Glu
225                 230                 235                 240

Asn Val Pro Pro Val Leu His Ile Thr Asn Thr Ala Thr Thr Val Leu
                245                 250                 255

Leu Asp Glu Phe Gly Val Gly Pro Leu Cys Lys Gly Asp Asn Leu Tyr
            260                 265                 270

Leu Ser Ala Val Asp Val Cys Gly Met Phe Thr Asn Arg Ser Gly Ser
        275                 280                 285

Gln Gln Trp Arg Gly Leu Ser Arg Tyr Phe Lys Val Gln Leu Arg Lys
    290                 295                 300

Arg Arg Val Lys Asn Pro Tyr Pro Ile Ser Phe Leu Leu Thr Asp Leu
305                 310                 315                 320

Ile Asn Arg Arg Thr Pro Arg Val Asp Gly Gln Pro Met Tyr Gly Met
                325                 330                 335

Asp Ala Gln Val Glu Glu Val Arg Val Phe Glu Gly Thr Glu Glu Leu
            340                 345                 350

Pro Gly Asp Pro Asp Met Met Arg Tyr Val Asp Lys Tyr Gly Gln Leu
        355                 360                 365

Gln Thr Lys Met Cys Ile Thr Gly Asp Ala Leu Val Ala Leu Pro Glu
    370                 375                 380

Gly Glu Ser Val Arg Ile Ala Asp Ile Val Pro Gly Ala Arg Pro Asn
385                 390                 395                 400

Ser Asp Asn Ala Ile Asp Leu Lys Val Leu Asp Arg His Gly Asn Pro
                405                 410                 415

Val Leu Ala Asp Arg Leu Phe His Ser Gly Glu His Pro Val Tyr Thr
            420                 425                 430

Val Arg Thr Val Glu Gly Leu Arg Val Thr Gly Thr Ala Asn His Pro
        435                 440                 445

Leu Leu Cys Leu Val Asp Val Ala Gly Val Pro Thr Leu Leu Trp Lys
    450                 455                 460

Leu Ile Asp Glu Ile Lys Pro Gly Asp Tyr Ala Val Ile Gln Arg Ser
465                 470                 475                 480

Ala Phe Ser Val Asp Cys Ala Gly Phe Ala Arg Gly Lys Pro Glu Phe
                485                 490                 495

Ala Pro Thr Thr Tyr Thr Val Gly Val Pro Gly Leu Val Arg Phe Leu
            500                 505                 510

Glu Ala His His Arg Asp Pro Asp Ala Gln Ala Ile Ala Asp Glu Leu
        515                 520                 525

Thr Asp Gly Arg Phe Tyr Tyr Ala Lys Val Ala Ser Val Thr Asp Ala
    530                 535                 540

Gly Val Gln Pro Val Tyr Ser Leu Arg Val Asp Thr Ala Asp His Ala
545                 550                 555                 560

Phe Ile Thr Asn Gly Phe Val Ser His Ala Thr Gly Leu Thr Gly Leu
                565                 570                 575

Asn Ser Gly Leu Thr Thr Asn Pro Gly Val Ser Ala Trp Gln Val Asn
            580                 585                 590

Thr Ala Tyr Thr Ala Gly Gln Leu Val Thr Tyr Asn Gly Lys Thr Tyr
        595                 600                 605

Lys Cys Leu Gln Pro His Thr Ser Leu Ala Gly Trp Glu Pro Ser Asn
    610                 615                 620

Val Pro Ala Leu Trp Gln Leu Gln
625                 630

<210> 15
<211> 632
<212> PRT
<213> synthetic

<400> 15

Met Ala Pro Thr Lys Arg Lys Gly Glu Pro Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Asp Asn Gly Ala Gly Lys Pro Val Gln Gly Thr Ser Phe His Phe
    130                 135                 140

Phe Ser Val Gly Gly Glu Ala Leu Glu Leu Gln Gly Val Val Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Arg Arg Arg Ser Gly Cys
            260                 265                 270

Gly Asn Lys Arg Thr Arg Gly Cys Gly Ser Glu Glu Glu Glu Gly Ser
        275                 280                 285

Gln Gln Trp Arg Gly Leu Ser Arg Tyr Phe Lys Val Gln Leu Arg Lys
    290                 295                 300

Arg Arg Val Lys Asn Pro Tyr Pro Ile Ser Phe Leu Leu Thr Asp Leu
305                 310                 315                 320

Ile Asn Arg Arg Thr Pro Arg Val Asp Gly Gln Pro Met Tyr Gly Met
                325                 330                 335

Asp Ala Gln Val Glu Glu Val Arg Val Phe Glu Gly Thr Glu Glu Leu
            340                 345                 350

Pro Gly Asp Pro Asp Met Met Arg Tyr Val Asp Lys Tyr Gly Gln Leu
        355                 360                 365

Gln Thr Lys Met Cys Ile Thr Gly Asp Ala Leu Val Ala Leu Pro Glu
    370                 375                 380

Gly Glu Ser Val Arg Ile Ala Asp Ile Val Pro Gly Ala Arg Pro Asn
385                 390                 395                 400

Ser Asp Asn Ala Ile Asp Leu Lys Val Leu Asp Arg His Gly Asn Pro
                405                 410                 415

Val Leu Ala Asp Arg Leu Phe His Ser Gly Glu His Pro Val Tyr Thr
            420                 425                 430

Val Arg Thr Val Glu Gly Leu Arg Val Thr Gly Thr Ala Asn His Pro
        435                 440                 445

Leu Leu Cys Leu Val Asp Val Ala Gly Val Pro Thr Leu Leu Trp Lys
    450                 455                 460

Leu Ile Asp Glu Ile Lys Pro Gly Asp Tyr Ala Val Ile Gln Arg Ser
465                 470                 475                 480

Ala Phe Ser Val Asp Cys Ala Gly Phe Ala Arg Gly Lys Pro Glu Phe
                485                 490                 495

Ala Pro Thr Thr Tyr Thr Val Gly Val Pro Gly Leu Val Arg Phe Leu
            500                 505                 510

Glu Ala His His Arg Asp Pro Asp Ala Gln Ala Ile Ala Asp Glu Leu
        515                 520                 525

Thr Asp Gly Arg Phe Tyr Tyr Ala Lys Val Ala Ser Val Thr Asp Ala
    530                 535                 540

Gly Val Gln Pro Val Tyr Ser Leu Arg Val Asp Thr Ala Asp His Ala
545                 550                 555                 560

Phe Ile Thr Asn Gly Phe Val Ser His Ala Thr Gly Leu Thr Gly Leu

565                 570                 575

Asn Ser Gly Leu Thr Thr Asn Pro Gly Val Ser Ala Trp Gln Val Asn
            580                 585                 590

Thr Ala Tyr Thr Ala Gly Gln Leu Val Thr Tyr Asn Gly Lys Thr Tyr
        595                 600                 605

Lys Cys Leu Gln Pro His Thr Ser Leu Ala Gly Trp Glu Pro Ser Asn
    610                 615                 620

Val Pro Ala Leu Trp Gln Leu Gln
625                 630

<210>  16
<211>  1065
<212>  DNA
<213>  JC-Polyomavirus

<400>  16
atggctccca ccaagcgcaa gggcgagccc aaggaccccg tgcaagtgcc caagctgctg      60

atccgtggtg gtgtcgaggt gctggaagtc aagaccggcg tggactccat taccgaggtg     120

gagtgcttcc tcacccccga gatgggtgac cctgacgagc acctgagggg cttctccaag     180

tccatctcca tctccgacac cttcgagtcc gactccccca accgtgacat gctgccctgc     240

tactccgtgg ctcgtatccc cctgcccaac ctgaacgagg acctgacttg cggcaacatc     300

ctgatgtggg aggctgtgac cctcaagacc gaggtcatcg gcgtgacttc cctgatgaac     360

gtgcactcca acggccaggc tacccacgac aacggtgctg gcaagcccgt gcagggaacc     420

tccttccact tcttctccgt gggtggcgag gctctggaac tccagggcgt ggtgttcaac     480

taccgtacca agtaccccga cggcaccatc ttccccaaga acgctactgt gcagtcccaa     540

gtgatgaaca ccgagcacaa ggcttacctg gacaagaaca aggcctaccc cgtggagtgc     600

tgggtgcccg accccacccg taacgagaac acccgttact tcggcaccct gaccggtgga     660

gagaacgtgc cccccgtgct gcacatcacc aacaccgcta ccaccgtgct gctggacgag     720

ttcggtgtcg gtcccctgtg caagggcgac aacctgtacc tgtccgctgt ggacgtgtgc     780

ggcatgttca ccaaccgttc cggttcccag cagtggcgtg gcctgtcccg ctacttcaag     840

gtgcagctgc gcaagcgtcg tgtgaagaac ccctacccta tctccttcct gctgaccgac     900

ctgatcaacc gtcgtacccc tcgtgtggac ggccagccca tgtacggcat ggacgctcag     960

gtggaagagg tccgcgtgtt cgagggcacc gaggaattgc ccggcgaccc cgacatgatg    1020

cgttacgtgg acaagtacgg ccagctccag accaagatgc tgtaa                    1065

<210>  17

<211>  63
<212>  DNA
<213>  synthetic

<400>  17
cgtagacgta gatcaggttg tggtaacaag cgaactagag ggtgcggatc cgaagaggaa      60

gag                                                                    63


<210>  18
<211>  1899
<212>  DNA
<213>  synthetic

<400>  18
atggctccca ccaagcgcaa gggcgagccc aaggaccccg tgcaagtgcc caagctgctg      60

atccgtggtg gtgtcgaggt gctggaagtc aagaccggcg tggactccat taccgaggtg     120

gagtgcttcc tcacccccga gatgggtgac cctgacgagc acctgagggg cttctccaag     180

tccatctcca tctccgacac cttcgagtcc gactccccca accgtgacat gctgccctgc     240

tactccgtgg ctcgtatccc cctgcccaac ctgaacgagg acctgacttg cggcaacatc     300

ctgatgtggg aggctgtgac cctcaagacc gaggtcatcg gcgtgacttc cctgatgaac     360

gtgcactcca acggccaggc tacccaccgt agacgtagat caggttgtgg taacaagcga     420

actagagggt gcggatccga agaggaagag ggtgctggca agcccgtgca gggaacctcc     480

ttccacttct tctccgtggg tggcgaggct ctggaactcc agggcgtggt gttcaactac     540

cgtaccaagt accccgacgg caccatcttc cccaagaacg ctactgtgca gtcccaagtg     600

atgaacaccg agcacaaggc ttacctggac aagaacaagg cctaccccgt ggagtgctgg     660

gtgcccgacc ccacccgtaa cgagaacacc cgttacttcg gcaccctgac cggtggagag     720

aacgtgcccc ccgtgctgca catcaccaac accgctacca ccgtgctgct ggacgagttc     780

ggtgtcggtc ccctgtgcaa gggcgacaac ctgtacctgt ccgctgtgga cgtgtgcggc     840

atgttcacca accgttccgg ttcccagcag tggcgtggcc tgtcccgcta cttcaaggtg     900

cagctgcgca agcgtcgtgt gaagaacccc taccctatct ccttcctgct gaccgacctg     960

atcaaccgtc gtacccctcg tgtggacggc cagcccatgt acggcatgga cgctcaggtg    1020

gaagaggtcc gcgtgttcga gggcaccgag gaattgcccg gcgaccccga catgatgcgt    1080

tacgtggaca agtacggcca gctccagacc aagatgtgca tcacgggaga tgcactagtt    1140

gccctacccg agggcgagtc ggtacgcatc gccgacatcg tgccgggtgc gcggcccaac    1200

agtgacaacg ccatcgacct gaaagtcctt gaccggcatg gcaatcccgt gctcgccgac    1260

cggctgttcc actccggcga gcatccggtg tacacggtgc gtacggtcga aggtctgcgt    1320

gtgacgggca ccgcgaacca cccgttgttg tgtttggtcg acgtcgccgg ggtgccgacc    1380

ctgctgtgga agctgatcga cgaaatcaag ccgggcgatt acgcggtgat tcaacgcagc    1440

gcattcagcg tcgactgtgc aggttttgcc cgcgggaaac ccgaatttgc gcccacaacc 1500

tacacagtcg gcgtccctgg actggtgcgt ttcttggaag cacaccaccg agacccggac 1560

gcccaagcta tcgccgacga gctgaccgac gggcggttct actacgcgaa agtcgccagt 1620

gtcaccgacg ccggcgtgca gccggtgtat agccttcgtg tcgacacggc agaccacgcg 1680

tttatcacga acgggttcgt cagccacgct actggcctca ccggtctgaa ctcaggcctc 1740

acgacaaatc ctggtgtatc cgcttggcag gtcaacacag cttatactgc gggacaattg 1800

gtcacatata acggcaagac gtataaatgt ttgcagcccc acacctcctt ggcaggatgg 1860

gaaccatcca acgttcctgc cttgtggcag cttcaatga 1899

<210> 19
<211> 1899
<212> DNA
<213> synthetic

<400> 19
atggctccca ccaagcgcaa gggcgagccc aaggaccccg tgcaagtgcc caagctgctg 60

atccgtggtg gtgtcgaggt gctggaagtc aagaccggcg tggactccat taccgaggtg 120

gagtgcttcc tcacccccga gatgggtgac cctgacgagc acctgagggg cttctccaag 180

tccatctcca tctccgacac cttcgagtcc gactccccca accgtgacat gctgccctgc 240

tactccgtgg ctcgtatccc cctgcccaac ctgaacgagg acctgacttg cggcaacatc 300

ctgatgtggg aggctgtgac cctcaagacc gaggtcatcg gcgtgacttc cctgatgaac 360

gtgcactcca acggccaggc tacccacgac aacggtgctg gcaagcccgt gcagggaacc 420

tccttccact tcttctccgt gggtggcgag gctctggaac tccagggcgt ggtgttcaac 480

taccgtacca agtaccccga cggcaccatc ttccccaaga acgctactgt gcagtcccaa 540

gtgatgaaca ccgagcacaa ggcttacctg gacaagaaca aggcctaccc cgtggagtgc 600

tgggtgcccg accccacccg taacgagaac acccgttact tcggcaccct gaccggtgga 660

gagaacgtgc cccccgtgct gcacatcacc aacaccgcta ccaccgtgct gctggacgag 720

ttcggtgtcg gtcccctgtg caagggcgac aacctgtacc tgtccgctgt ggacgtgtgc 780

ggcatgttca ccaaccgtag acgtagatca ggttgtggta acaagcgaac tagagggtgc 840

ggatccgaag aggaagaggg ttcccagcag tggcgtggcc tgtcccgcta cttcaaggtg 900

cagctgcgca agcgtcgtgt gaagaacccc taccctatct ccttcctgct gaccgacctg 960

atcaaccgtc gtacccctcg tgtggacggc cagcccatgt acggcatgga cgctcaggtg 1020

gaagaggtcc gcgtgttcga gggcaccgag gaattgcccg gcgaccccga catgatgcgt 1080

tacgtggaca agtacggcca gctccagacc aagatgtgca tcacgggaga tgcactagtt 1140

gccctacccg agggcgagtc ggtacgcatc gccgacatcg tgccgggtgc gcggcccaac 1200

agtgacaacg ccatcgacct gaaagtcctt gaccggcatg gcaatcccgt gctcgccgac 1260

cggctgttcc actccggcga gcatccggtg tacacggtgc gtacggtcga aggtctgcgt 1320

gtgacgggca ccgcgaacca cccgttgttg tgtttggtcg acgtcgccgg ggtgccgacc 1380

ctgctgtgga agctgatcga cgaaatcaag ccgggcgatt acgcggtgat tcaacgcagc 1440

gcattcagcg tcgactgtgc aggttttgcc cgcgggaaac ccgaatttgc gcccacaacc 1500

tacacagtcg gcgtccctgg actggtgcgt ttcttggaag cacaccaccg agacccggac 1560

gcccaagcta tcgccgacga gctgaccgac gggcggttct actacgcgaa agtcgccagt 1620

gtcaccgacg ccggcgtgca gccggtgtat agccttcgtg tcgacacggc agaccacgcg 1680

tttatcacga acgggttcgt cagccacgct actggcctca ccggtctgaa ctcaggcctc 1740

acgacaaatc ctggtgtatc cgcttggcag gtcaacacag cttatactgc gggacaattg 1800

gtcacatata acggcaagac gtataaatgt ttgcagcccc acacctcctt ggcaggatgg 1860

gaaccatcca acgttcctgc cttgtggcag cttcaatga 1899

<210> 20
<211> 7
<212> PRT
<213> synthetic

<400> 20

Arg Arg Arg Arg Ser Gly Cys
1               5

<210> 21
<211> 7
<212> PRT
<213> synthetic

<400> 21

Cys Gly Ser Glu Glu Glu Glu
1               5

<210> 22
<211> 342
<212> PRT
<213> JC-Polyomavirus

<400> 22

Met Gly Ala Ala Leu Ala Leu Leu Gly Asp Leu Val Ala Thr Val Ser
1               5                   10                  15

Glu Ala Ala Ala Ala Thr Gly Phe Ser Val Ala Glu Ile Ala Ala Gly
            20                  25                  30

Glu Ala Ala Ala Thr Ile Glu Val Glu Ile Ala Ser Leu Ala Thr Val
        35                  40                  45

Glu Gly Ile Thr Ser Thr Ser Glu Ala Ile Ala Ala Ile Gly Leu Thr
    50                  55                  60

Pro Glu Thr Tyr Ala Val Ile Thr Gly Ala Pro Gly Ala Val Ala Gly
65                  70                  75                  80

Phe Ala Ala Leu Val Gln Thr Val Thr Gly Gly Ser Ala Ile Ala Gln
                85                  90                  95

Leu Gly Tyr Arg Phe Phe Ala Asp Trp Asp His Lys Val Ser Thr Val
            100                 105                 110

Gly Leu Phe Gln Gln Pro Ala Met Ala Leu Gln Leu Phe Asn Pro Glu
        115                 120                 125

Asp Tyr Tyr Asp Ile Leu Phe Pro Gly Val Asn Ala Phe Val Asn Asn
    130                 135                 140

Ile His Tyr Leu Asp Pro Arg His Trp Gly Pro Ser Leu Phe Ser Thr
145                 150                 155                 160

Ile Ser Gln Ala Phe Trp Asn Leu Val Arg Asp Asp Leu Pro Ala Leu
                165                 170                 175

Thr Ser Gln Glu Ile Gln Arg Arg Thr Gln Lys Leu Phe Val Glu Ser
            180                 185                 190

Leu Ala Arg Phe Leu Glu Glu Thr Thr Trp Ala Ile Val Asn Ser Pro
        195                 200                 205

Ala Asn Leu Tyr Asn Tyr Ile Ser Asp Tyr Tyr Ser Arg Leu Ser Pro
    210                 215                 220

Val Arg Pro Ser Met Val Arg Gln Val Ala Gln Arg Glu Gly Thr Tyr
225                 230                 235                 240

Ile Ser Phe Gly His Ser Tyr Thr Gln Ser Ile Asp Asp Ala Asp Ser
                245                 250                 255

Ile Gln Glu Val Thr Gln Arg Leu Asp Leu Lys Thr Pro Asn Val Gln
            260                 265                 270

Ser Gly Glu Phe Ile Glu Arg Ser Ile Ala Pro Gly Gly Ala Asn Gln
        275                 280                 285

Arg Ser Pro Gln Trp Met Leu Pro Leu Leu Leu Gly Leu Tyr Gly Thr
    290                 295                 300

Val Thr Pro Ala Leu Glu Ala Tyr Glu Asp Gly Pro Asn Lys Lys Lys
305                 310                 315                 320

Arg Arg Lys Glu Gly Pro Arg Ala Ser Lys Thr Ser Tyr Lys Arg Arg
                325                 330                 335

Ser Arg Ser Ser Arg Ser
            340

<210>  23
<211>  225
<212>  PRT
<213>  JC-Polyomavirus

<400>  23

Met Ala Leu Gln Leu Phe Asn Pro Glu Asp Tyr Tyr Asp Ile Leu Phe
1               5                   10                  15

Pro Gly Val Asn Ala Phe Val Asn Asn Ile His Tyr Leu Asp Pro Arg
            20                  25                  30

His Trp Gly Pro Ser Leu Phe Ser Thr Ile Ser Gln Ala Phe Trp Asn
        35                  40                  45

Leu Val Arg Asp Asp Leu Pro Ala Leu Thr Ser Gln Glu Ile Gln Arg
    50                  55                  60

Arg Thr Gln Lys Leu Phe Val Glu Ser Leu Ala Arg Phe Leu Glu Glu
65                  70                  75                  80

Thr Thr Trp Ala Ile Val Asn Ser Pro Ala Asn Leu Tyr Asn Tyr Ile
                85                  90                  95

Ser Asp Tyr Tyr Ser Arg Leu Ser Pro Val Arg Pro Ser Met Val Arg
            100                 105                 110

Gln Val Ala Gln Arg Glu Gly Thr Tyr Ile Ser Phe Gly His Ser Tyr
        115                 120                 125

Thr Gln Ser Ile Asp Asp Ala Asp Ser Ile Gln Glu Val Thr Gln Arg
    130                 135                 140

Leu Asp Leu Lys Thr Pro Asn Val Gln Ser Gly Glu Phe Ile Glu Arg
145                 150                 155                 160

Ser Ile Ala Pro Gly Gly Ala Asn Gln Arg Ser Ala Pro Gln Trp Met
                165                 170                 175

Leu Pro Leu Leu Leu Gly Leu Tyr Gly Thr Val Thr Pro Ala Leu Glu
            180                 185                 190

Ala Tyr Glu Asp Gly Pro Asn Lys Lys Lys Arg Arg Lys Glu Gly Pro
        195                 200                 205

Arg Ala Ser Ser Lys Thr Ser Tyr Lys Arg Arg Ser Arg Ser Ser Arg
    210                 215                 220

Ser
225

<210>  24
<211>  36
<212>  PRT
<213>  JC-Polyomavirus

<400>  24

Ser Gly Glu Phe Ile Glu Arg Ser Ile Ala Pro Gly Gly Ala Asn Gln
1               5                   10                  15

Arg Ser Ala Pro Gln Trp Met Leu Pro Leu Leu Leu Gly Leu Tyr Gly
            20                  25                  30

Thr Val Thr Pro
        35

<210>  25
<211>  51
<212>  PRT
<213>  Homo sapiens (human)

<400>  25

Met Ala Arg Tyr Arg Cys Cys Arg Ser Gln Ser Arg Ser Arg Tyr Tyr
1               5                   10                  15

Arg Gln Arg Gln Arg Ser Arg Arg Arg Arg Arg Arg Ser Cys Gln Thr
            20                  25                  30

Arg Arg Arg Ala Met Arg Cys Cys Arg Pro Arg Tyr Arg Pro Arg Cys
        35                  40                  45

Arg Arg His
    50

<210>  26
<211>  21
<212>  PRT
<213>  Homo sapiens (human)

<400>  26

Ser Gln Ser Arg Ser Arg Tyr Tyr Arg Gln Arg Gln Arg Ser Arg Arg
1               5                   10                  15

Arg Arg Arg Arg Ser
            20

<210>  27
<211>  17
<212>  PRT
<213>  Human immunodeficiency virus (HIV)

<400>  27

Gly Arg Lys Lys Arg Arg Gln Arg Arg Arg Pro Pro Gln Lys Arg Lys
1               5                   10                  15

Gly

<210>  28
<211>  16
<212>  PRT
<213>  Antennapedia spec.

<400>  28

Arg Gln Ile Lys Ile Trp Phe Gln Asn Arg Arg Met Lys Trp Lys Lys
1               5                   10                  15

<210>  29
<211>  21
<212>  PRT
<213>  Human papillomavirus type 33 (HPV33)

<400>  29

Phe Ile Leu Arg Arg Arg Arg Lys Arg Phe Pro Tyr Phe Phe Thr Asp
1               5                   10                  15

Val Arg Val Ala Ala
            20

<210>  30
<211>  21
<212>  PRT
<213>  Human papillomavirus type 33 (HPV33)

<400>  30

Asp Asp Leu Arg Arg Arg Arg Lys Arg Phe Pro Tyr Phe Phe Thr Asp
1               5                   10                  15

Val Arg Val Ala Ala
            20

<210>  31
<211>  18
<212>  PRT
<213>  synthetic

<400>  31

Arg Gly Gly Arg Leu Ser Tyr Ser Arg Arg Arg Phe Ser Thr Ser Thr
1               5                   10                  15

Gly Arg

<210>  32
<211>  10
<212>  PRT
<213>  synthetic

<400>  32

Arg Arg Leu Ser Tyr Ser Arg Arg Arg Phe
1               5                   10

<210>  33
<211>  12
<212>  PRT
<213>  synthetic

<400>  33

Pro Ile Arg Arg Arg Lys Lys Leu Arg Arg Leu Lys
1               5                   10

<210>  34
<211>  12
<212>  PRT
<213>  synthetic

<400>  34

Arg Arg Gln Arg Arg Thr Ser Lys Leu Met Lys Arg
1               5                   10

<210>  35
<211>  15
<212>  PRT
<213>  FHV

<400>  35

Arg Arg Arg Arg Asn Arg Thr Arg Arg Asn Arg Arg Arg Val Arg
1               5                   10                  15

<210>  36
<211>  19
<212>  PRT
<213>  BMV

<400>  36

Lys Met Thr Arg Ala Gln Arg Arg Ala Ala Ala Arg Arg Asn Arg Trp
1               5                   10                  15

Thr Ala Arg

<210>  37
<211>  13
<212>  PRT
<213>  synthetic

<400>  37

Thr Arg Arg Gln Arg Thr Arg Arg Ala Arg Arg Asn Arg
1               5                   10

<210>  38
<211>  13
<212>  PRT
<213>  Human immunodeficiency virus (HIV)

<400>  38

Gly Arg Lys Lys Arg Arg Gln Arg Arg Arg Pro Pro Gln
1               5                   10

<210>  39
<211>  13
<212>  PRT
<213>  Human papillomavirus type 33 (HPV33)

<400>  39

Gly Arg Arg Arg Arg Arg Arg Arg Arg Arg Pro Pro Gln
1               5                   10

<210>  40
<211>  47
<212>  DNA
<213>  synthetic

<400>  40
gaactagagg gtgcggatcc gaagaggaag agggttccca gcagtgg                    47

<210>  41
<211>  45

<212>  DNA
<213>  synthetic

<400>  41
gcttgttacc acaacctgat ctacgtctac ggttggtgaa catgc                    45


<210>  42
<211>  7714
<212>  DNA
<213>  synthetic

<400>  42
aactacgtca ggtggcactt ttcggggaaa tgtgcgcgga acccctattt gtttattttt      60

ctaaatacat tcaaatatgt atccgctcat gagacaataa ccctgataaa tgcttcaata     120

atattgaaaa aggaagagta tgagtattca acatttccgt gtcgccctta ttcccttttt     180

tgcggcattt tgccttcctg tttttgctca cccagaaacg ctggtgaaag taaaagatgc     240

tgaagatcag ttgggtgcac gagtgggtta catcgaactg gatctcaaca gcggtaagat     300

ccttgagagt tttcgccccg aagaacgttc tccaatgatg agcactttta aagttctgct     360

atgtggcgcg gtattatccc gtgttgacgc cgggcaagag caactcggtc gccgcataca     420

ctattctcag aatgacttgg ttgagtactc accagtcaca gaaaagcatc ttacggatgg     480

catgacagta agagaattat gcagtgctgc cataaccatg agtgataaca ctgcggccaa     540

cttacttctg acaacgatcg gaggaccgaa ggagctaacc gcttttttgc acaacatggg     600

ggatcatgta actcgccttg atcgttggga accggagctg aatgaagcca taccaaacga     660

cgagcgtgac accacgatgc ctgtagcaat ggcaacaacg ttgcgcaaac tattaactgg     720

cgaactactt actctagctt cccggcaaca attaatagac tggatggagg cggataaagt     780

tgcaggacca cttctgcgct cggcccttcc ggctggctgg tttattgctg ataaatctgg     840

agccggtgag cgtgggtctc gcggtatcat tgcagcactg gggccagatg gtaagccctc     900

ccgtatcgta gttatctaca cgacggggag tcaggcaact atggatgaac gaaatagaca     960

gatcgctgag ataggtgcct cactgattaa gcattggtaa ctgtcagacc aagtttactc    1020

atatatactt tagattgatt taccccggtt gataatcaga aaagccccaa aaacaggaag    1080

attgtataag caaatattta aattgtaaac gttaatattt tgttaaaatt cgcgttaaat    1140

ttttgttaaa tcagctcatt ttttaaccaa taggccgaaa tcggcaaaat cccttataaa    1200

tcaaaagaat agcccgagat agggttgagt gttgttccag tttggaacaa gagtccacta    1260

ttaaagaacg tggactccaa cgtcaaaggg cgaaaaaccg tctatcaggg cgatggccca    1320

ctacgtgaac catcacccaa atcaagtttt ttggggtcga ggtgccgtaa agcactaaat    1380

cggaacccta aagggagccc ccgatttaga gcttgacggg gaaagccggc gaacgtggcg    1440

agaaaggaag ggaagaaagc gaaaggagcg ggcgctaggg cgctggcaag tgtagcggtc    1500

acgctgcgcg taaccaccac acccgccgcg cttaatgcgc cgctacaggg cgcgtaaaag 1560

gatctaggtg aagatccttt ttgataatct catgaccaaa atcccttaac gtgagttttc 1620

gttccactga gcgtcagacc ccgtagaaaa gatcaaagga tcttcttgag atcctttttt 1680

tctgcgcgta atctgctgct tgcaaacaaa aaaaccaccg ctaccagcgg tggtttgttt 1740

gccggatcaa gagctaccaa ctctttttcc gaaggtaact ggcttcagca gagcgcagat 1800

accaaatact gtccttctag tgtagccgta gttaggccac cacttcaaga actctgtagc 1860

accgcctaca tacctcgctc tgctaatcct gttaccagtg gctgctgcca gtggcgataa 1920

gtcgtgtctt accgggttgg actcaagacg atagttaccg gataaggcgc agcggtcggg 1980

ctgaacgggg ggttcgtgca cacagcccag cttggagcga acgacctaca ccgaactgag 2040

atacctacag cgtgagctat gagaaagcgc cacgcttccc gaagggagaa aggcggacag 2100

gtatccggta agcggcaggg tcggaacagg agagcgcacg agggagcttc cagggggaaa 2160

cgcctggtat ctttatagtc ctgtcgggtt tcgccacctc tgacttgagc gtcgattttt 2220

gtgatgctcg tcaggggggc ggagcctatg gaaaaacgcc agcaacgcgg cctttttacg 2280

gttcctggcc ttttgctggc cttttgctca catgttcttt cctgcgttat cccctgattc 2340

tgtggataac cgtattaccg cctttgagtg agctgatacc gctcgccgca gccgaacgac 2400

cgagcgcagc gagtcagtga gcgaggaagc tatggtgcac tctcagtaca atctgctctg 2460

atgccgcata gttaagccag tatacactcc gctatcgcta cgtgactggg tcatggctgc 2520

gccccgacac ccgccaacac ccgctgacgc gccctgacgg gcttgtctgc tcccggcatc 2580

cgcttacaga caagctgtga ccgtctccgg gagctgcatg tgtcagaggt tttcaccgtc 2640

atcaccgaaa cgcgcgaggc agctgcggta aagctcatca gcgtggtcgt gcagcgattc 2700

acagatgtct gcctgttcat ccgcgtccag ctcgttgagt ttctccagaa gcgttaatgt 2760

ctggcttctg ataaagcggg ccatgttaag ggcggttttt tcctgtttgg tcactgatgc 2820

ctccgtgtaa gggggatttc tgttcatggg ggtaatgata ccgatgaaac gagagaggat 2880

gctcacgata cgggttactg atgatgaaca tgcccggtta ctggaacgtt gtgagggtaa 2940

acaactggcg gtatggatgc ggcgggacca gagaaaaatc actcagggtc aatgccagcc 3000

gaacgccagc aagacgtagc ccagcgcgtc ggccgccatg ccggcgataa tggcctgctt 3060

ctcgccgaaa cgtttggtgg cgggaccagt gacgaaggct tgagcgaggg cgtgcaagat 3120

tccgaatacc gcaagcgaca ggccgatcat cgtcgcgctc cagcgaaagc ggtcctcgcc 3180

gaaaatgacc cagagcgctg ccggcacctg tcctacgagt tgcatgataa agaagacagt 3240

cataagtgcg gcgacgatag tcatgccccg cgcccaccgg aaggagctga ctgggttgaa 3300

ggctctcaag ggcatcggtc gagatcccgg tgcctaatga gtgagctaac ttacattaat 3360

tgcgttgcgc tcactgcccg ctttccagtc gggaaacctg tcgtgccagc tgcattaatg 3420

aatcggccaa cgcgcgggga gaggcggttt gcgtattggg cgccagggtg gtttttcttt 3480

tcaccagtga gacgggcaac agctgattgc ccttcaccgc ctggccctga gagagttgca 3540

gcaagcggtc cacgctggtt tgccccagca ggcgaaaatc ctgtttgatg gtggttaacg 3600

gcgggatata acatgagctg tcttcggtat cgtcgtatcc cactaccgag atatccgcac 3660

caacgcgcag cccggactcg gtaatggcgc gcattgcgcc cagcgccatc tgatcgttgg 3720

caaccagcat cgcagtggga acgatgccct cattcagcat ttgcatggtt tgttgaaaac 3780

cggacatggc actccagtcg ccttcccgtt ccgctatcgg ctgaatttga ttgcgagtga 3840

gatatttatg ccagccagcc agacgcagac gcgccgagac agaacttaat gggcccgcta 3900

acagcgcgat ttgctggtga cccaatgcga ccagatgctc cacgcccagt cgcgtaccgt 3960

cttcatggga gaaaataata ctgttgatgg gtgtctggtc agagacatca agaaataacg 4020

ccggaacatt agtgcaggca gcttccacag caatggcatc ctggtcatcc agcggatagt 4080

taatgatcag cccactgacg cgttgcgcga gaagattgtg caccgccgct ttacaggctt 4140

cgacgccgct tcgttctacc atcgacacca ccacgctggc acccagttga tcggcgcgag 4200

atttaatcgc cgcgacaatt tgcgacggcg cgtgcagggc cagactggag gtggcaacgc 4260

caatcagcaa cgactgtttg cccgccagtt gttgtgccac gcggttggga atgtaattca 4320

gctccgccat cgccgcttcc actttttccc gcgttttcgc agaaacgtgg ctggcctggt 4380

tcaccacgcg ggaaacggtc tgataagaga caccggcata ctctgcgaca tcgtataacg 4440

ttactggttt cacattcacc accctgaatt gactctcttc cgggcgctat catgccatac 4500

cgcgaaaggt tttgcgccat tcgatggtgt ccgggatctc gacgctctcc cttatgcgac 4560

tcctgcatta ggaagcagcc cagtagtagg ttgaggccgt tgagcaccgc cgccgcaagg 4620

aatggtgcat gccggcatgc cgccctttcg tcttcaagaa ttaattccca attccccagg 4680

catcaaataa aacgaaaggc tcagtcgaaa gactgggcct ttcgttttat ctgttgtttg 4740

tcggtgaacg ctctcctgag taggacaaat ccgccgggag cggatttgaa cgttgcgaag 4800

caacggcccg gagggtggcg ggcaggacgc ccgccataaa ctgccaggaa ttaattcccc 4860

aggcatcaaa taaaacgaaa ggctcagtcg aaagactggg cctttcgttt tatctgttgt 4920

ttgtcggtga acgctctcct gagtaggaca aatccgccgg gagcggattt gaacgttgcg 4980

aagcaacggc ccggagggtg gcgggcagga cgcccgccat aaactgccag gaattaattc 5040

cccaggcatc aaataaaacg aaaggctcag tcgaaagact gggcctttcg ttttatctgt 5100

tgtttgtcgg tgaacgctct cctgagtagg acaaatccgc cgggagcgga tttgaacgtt 5160

gcgaagcaac ggcccggagg gtggcgggca ggacgcccgc cataaactgc caggaattaa 5220

ttccccaggc atcaaataaa acgaaaggct cagtcgaaag actgggcctt tcgttttatc 5280

tgttgtttgt cggtgaacgc tctcctgagt aggacaaatc cgccgggagc ggatttgaac 5340
gttgcgaagc aacggcccgg agggtggcgg gcaggacgcc cgccataaac tgccaggaat 5400
taattcccca ggcatcaaat aaaacgaaag gctcagtcga aagactgggc ctttcgtttt 5460
atctgttgtt tgtcggtgaa cgctctcctg agtaggacaa atccgccggg agcggatttg 5520
aacgttgcga agcaacggcc cggagggtgg cgggcaggac gcccgccata aactgccagg 5580
aattggggat cggaattaat tcccggttta aaccggggat ctcgatcccg cgaaattaat 5640
acgactcact ataggggaat tgtgagcgga taacaattcc cctctagaaa taattttgtt 5700
taactttaag aaggagatat acatatggct cccaccaagc gcaagggcga gcccaaggac 5760
cccgtgcaag tgcccaagct gctgatccgt ggtggtgtcg aggtgctgga agtcaagacc 5820
ggcgtggact ccattaccga ggtggagtgc ttcctcaccc ccgagatggg tgaccctgac 5880
gagcacctga ggggcttctc caagtccatc tccatctccg acaccttcga gtccgactcc 5940
cccaaccgtg acatgctgcc ctgctactcc gtggctcgta tccccctgcc caacctgaac 6000
gaggacctga cttgcggcaa catcctgatg tgggaggctg tgaccctcaa gaccgaggtc 6060
atcggcgtga cttccctgat gaacgtgcac tccaacggcc aggctaccca cgacaacggt 6120
gctggcaagc ccgtgcaggg aacctccttc cacttcttct ccgtgggtgg cgaggctctg 6180
gaactccagg gcgtggtgtt caactaccgt accaagtacc ccgacggcac catcttcccc 6240
aagaacgcta ctgtgcagtc ccaagtgatg aacaccgagc acaaggctta cctggacaag 6300
aacaaggcct accccgtgga gtgctgggtg cccgacccca cccgtaacga gaacacccgt 6360
tacttcggca ccctgaccgg tggagagaac gtgccccccg tgctgcacat caccaacacc 6420
gctaccaccg tgctgctgga cgagttcggt gtcggtcccc tgtgcaaggg cgacaacctg 6480
tacctgtccg ctgtggacgt gtgcggcatg ttcaccaacc gttccggttc ccagcagtgg 6540
cgtggcctgt cccgctactt caaggtgcag ctgcgcaagc gtcgtgtgaa gaacccctac 6600
cctatctcct tcctgctgac cgacctgatc aaccgtcgta cccctcgtgt ggacggccag 6660
cccatgtacg gcatggacgc tcaggtggaa gaggtccgcg tgttcgaggg caccgaggaa 6720
ttgcccggcg accccgacat gatgcgttac gtggacaagt acggccagct ccagaccaag 6780
atgtgcatca cgggagatgc actagttgcc ctacccgagg gcgagtcggt acgcatcgcc 6840
gacatcgtgc cgggtgcgcg gcccaacagt gacaacgcca tcgacctgaa agtccttgac 6900
cggcatggca atcccgtgct cgccgaccgg ctgttccact ccggcgagca tccggtgtac 6960
acggtgcgta cggtcgaagg tctgcgtgtg acgggcaccg cgaaccaccc gttgttgtgt 7020
ttggtcgacg tcgccggggt gccgaccctg ctgtggaagc tgatcgacga aatcaagccg 7080
ggcgattacg cggtgattca acgcagcgca ttcagcgtcg actgtgcagg ttttgcccgc 7140
gggaaacccg aatttgcgcc cacaacctac acagtcggcg tccctggact ggtgcgtttc 7200

ttggaagcac accaccgaga cccggacgcc caagctatcg ccgacgagct gaccgacggg 7260

cggttctact acgcgaaagt cgccagtgtc accgacgccg gcgtgcagcc ggtgtatagc 7320

cttcgtgtcg acacggcaga ccacgcgttt atcacgaacg ggttcgtcag ccacgctact 7380

ggcctcaccg gtctgaactc aggcctcacg acaaatcctg gtgtatccgc ttggcaggtc 7440

aacacagctt atactgcggg acaattggtc acatataacg gcaagacgta taaatgtttg 7500

cagccccaca cctccttggc aggatgggaa ccatccaacg ttcctgcctt gtggcagctt 7560

caatgactgc aggaagggga tccggctgct aacaaagccc gaaaggaagc tgagttggct 7620

gctgccaccg ctgagcaata actagcataa ccccttgggg cctctaaacg ggtcttgagg 7680

ggttttttgc tgaaaggagg aactatatcc ggat 7714

<210> 43
<211> 45
<212> DNA
<213> synthetic

<400> 43
gaactagagg gtgcggatcc gaagaggaag agggtgctgg caagc 45

<210> 44
<211> 45
<212> DNA
<213> synthetic

<400> 44
gcttgttacc acaacctgat ctacgtctac ggtgggtagc ctggc 45